(19) Europäisches Patentamt
European Patent Office
Office européen des brevets

(11) EP 2 358 338 B1

(12) **EUROPÄISCHE PATENTSCHRIFT**

(45) Veröffentlichungstag und Bekanntmachung des Hinweises auf die Patenterteilung:
**16.10.2013 Patentblatt 2013/42**

(21) Anmeldenummer: **09784068.0**

(22) Anmeldetag: **30.11.2009**

(51) Int Cl.:
***A61K 8/34*** (2006.01) ***A61K 8/49*** (2006.01)
***A61Q 5/10*** (2006.01)

(86) Internationale Anmeldenummer:
**PCT/EP2009/066019**

(87) Internationale Veröffentlichungsnummer:
**WO 2010/072511 (01.07.2010 Gazette 2010/26)**

(54) **FÄRBEMITTEL, ENTHALTEND SPEZIELLE HALOGENSUBSTITUIERTE BRENZCATECHINALDEHYDE UND SPEZIELLE CH-ACIDE VERBINDUNGEN**

DYE, COMPRISING SPECIAL HALOGEN SUBSTITUTED PYROCATECHOL ALDEHYDES AND SPECIAL CH-ACIDIC COMPOUNDS

PRODUIT COLORANT, CONTENANT DES ALDÉHYDES DE LA PYROCATÉCHINE SPÉCIAUX SUBSTITUÉS PAR UN HALOGÈNE ET DES COMPOSÉS CH-ACIDE SPÉCIAUX

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **15.12.2008 DE 102008061862**

(43) Veröffentlichungstag der Anmeldung:
**24.08.2011 Patentblatt 2011/34**

(73) Patentinhaber: **Henkel AG & Co. KGaA**
**40589 Düsseldorf (DE)**

(72) Erfinder:
- **GROß, Wibke**
  **41836 Hückelhoven (DE)**
- **NEMITZ, Ralph**
  **41363 Jüchen (DE)**
- **OBERKOBUSCH, Doris**
  **40591 Düsseldorf (DE)**
- **STEPHAN, Laura**
  **40589 Düsseldorf (DE)**

(56) Entgegenhaltungen:
**DE-A1-102004 044 231** **DE-A1-102005 062 834**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 358 338 B1

## Beschreibung

Die Erfindung betrifft ein Mittel zum Färben von keratinhaltigen Fasern, insbesondere menschlichen Haaren, das spezielle halogensubstituierte Brenzcatechinaldehyde in Kombination mit speziellen CH-aciden Verbindungen enthält, die Verwendung dieser Kombination in Mitteln zum Färben von keratinhaltigen Fasern, zur Farbauffrischung bzw. Nuancierung von bereits gefärbten keratinhaltigen Fasern sowie ein Verfahren zum Färben von keratinhaltigen Fasern, insbesondere menschlichen Haaren.

Zur Bereitstellung farbverändernder kosmetischer Mittel, insbesondere für die Haut oder keratinhaltige Fasern wie beispielsweise menschliche Haare, kennt der Fachmann je nach Anforderungen an die Färbung bzw. Farbveränderung diverse Systeme.

Sollen im Allgemeinen Substrate aufgehellt oder gar gebleicht werden, werden die das Substrat färbenden synthetischen und/oder natürlichen Farbstoffe meist oxidativ unter Einsatz von entsprechenden Oxidationsmitteln, wie beispielsweise Wasserstoffperoxid, entfärbt.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Als Entwicklerkomponenten werden üblicherweise primäre aromatische Amine mit einer weiteren, in para- oder ortho- Position befindlichen, freien oder substituierten Hydroxy- oder Aminogruppe, heterozyklische Hydrazone, Diaminopyrazolderivate sowie 2, 4, 5, 6- Tetraaminopyrimidin und dessen Derivate eingesetzt.

Als Kupplerkomponenten werden in der Regel m-Phenylendiaminderivate, Naphthole, Pyridinderivate, Resorcin und Resorcinderivate, Pyrazolone und m-Aminophenole verwendet.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Eine weitere Möglichkeit zur Farbveränderung bietet die Verwendung von Färbemitteln, welche sogenannte Oxofarbstoffvorprodukte enthalten. Eine erste Klasse der Oxofarbstoffvorprodukte sind Verbindungen mit mindestens einer reaktiven Carbonylgruppe, insbesondere einer Formylgruppe. Diese erste Klasse wird als Komponente (Oxo1) bezeichnet. Eine zweite Klasse der Oxofarbstoffvorprodukte bilden CH-acide Verbindungen und Verbindungen mit primärer oder sekundärer Aminogruppe oder Hydroxygruppe, die wiederum ausgewählt werden aus Verbindungen der Gruppe, die gebildet wird aus primären oder sekundären aromatischen Aminen, stickstoffhaltigen heterozyklischen Verbindungen sowie aromatischen Hydroxyverbindungen. Diese zweite Klasse wird als Komponente (Oxo2) bezeichnet. Die vorgenannten Komponenten (Oxo1) und (Oxo2) sind im Allgemeinen selbst keine Farbstoffe und eignen sich daher jede für sich genommen allein nicht zur Färbung keratinhaltiger Fasern. In Kombination bilden sie in einem nichtoxidativen Prozess der sogenannten Oxofärbung Farbstoffe aus. Die resultierenden Färbungen besitzen teilweise Farbechtheiten auf der keratinhaltigen Faser, die mit denen der Oxidationsfärbung vergleichbar sind.

Das mit der schonenden Oxofärbung erzielbare Nuancenspektrum ist sehr breit und die erhaltene Färbung weist oftmals eine akzeptable Brillanz und Farbtiefe auf. Unter Verbindungen der Komponente (Oxo2) können allerdings auch entsprechende Oxidationsfarbstoffvorprodukte vom Entwickler- und/oder Kupplertyp mit oder ohne Einsatz eines Oxidationsmittels Verwendung finden. Somit lässt sich die Methode der Oxofärbung ohne weiteres mit dem oxidativen Färbesystem kombinieren.

Die Palette käuflicher Haarfarben enthält neben den leuchtenden Modetönen auch eine große Vielfalt von

Naturtönen, welche insbesondere eine große Bandbreite von Blond- und Braunnuancen sowie Schwarz umfassen. Speziell für das Abdecken des ergrauten Haares und das Wiederherstellen der ursprünglichen Haarfarbe sind diese Naturtöne von Bedeutung.

Insbesondere zur Formulierung von natürlich wirkenden Haarfarbnuancen ist es von großer Wichtigkeit, mit einem Färbesystem das komplette Farbspektrum erzeugen zu können. Neben der Ausbildung von Rot- und Braunnuancen für Naturtöne und modische Kupfernuancen ist insbesondere die Schwarzfärbung von großer Wichtigkeit.

Bei Verwendung des Oxofärbesystems wird die Herstellung einer Schwarznuance meist durch die Abmischung verschiedener Farbnuancen erzielt. Hierbei ist es erforderlich, Farbstoffe mit Gelbkomponente, Farbstoffe mit einem Rotanteil sowie Farbstoffe mit Blauanteil zusammen zu verwenden. Bedingt durch diesen Mischungsprozess ist oft die Verfügbarkeit einer großen Anzahl von Farbstoffkomponenten erforderlich.

Das Anforderungsprofil an die erzeugten Nuancen umfasst neben einer umfassenden Farbpalette ein gutes Egalisiervermögen, um ein unterschiedliches Aufziehvermögen auf unterschiedlich geschädigte Teile des Haares und ein hieraus resultierendes uneinheitliches Farbergebnis zu vermeiden. Auch kann eine Nuance aufgrund verschieden guter Wasch- oder Lichtechtheiten der verwendeten unterschiedlichen Farbstoffe im Verlauf der Zeit Farbverschiebungen unterliegen, welche vom Verbraucher nicht gewünscht sind und daher bei der Entwicklung neuer Farbstoffkombinationen für Oxofärbesysteme unbedingt zu vermeiden sind.

Haarfärbesysteme, welche den Einsatz einer Kombination aus reaktiven Carbonylverbindungen, insbesondere Aldehyden und CH- aciden Verbindungen beanspruchen, sind dem Fachmann bereits aus der Patentliteratur bekannt. In den Dokumenten EP1534227, EP1778176, EP1789014, WO2006/119819, WO2007/079802 und WO2008/074578 sind Färbesysteme genannt, die Färbungen mit CH- aciden Verbindungen beschreiben.

Insbesondere direkt nach dem aus oben zitiertem Stand der Technik durchgeführten Färbeprozess werden stark leuchtende und brillante Nuancen erhalten.

Es liegt jedoch im grundlegenden Interesse des Anwenders, daß die im Zuge des Haarfärbeprozesses erhaltenen Färbungen nicht nur direkt nach der Anwendung optisch überzeugen, sondern auch dann noch ausreichend intensiv sind, wenn die Haare mehrmals gewaschen wurden oder anderen Umwelteinflüssen länger ausgesetzt waren. Bei dunklen Naturtönen und insbesondere bei Schwarznuancen wiesen die mit Hilfe dieses neuen Färbesystems erzeugten Nuancen bislang große Schwächen auf. Gerade bei diesen dunklen Nuancen war der durch Haarwäschen hervorgerufene Farbverlust besonders auffällig und oftmals mit Nuancenverschiebungen ins Blaue, Braune oder Gräuliche verbunden. Somit war es bislang nicht möglich, unter Einsatz einer aus dem Stand der Technik bekannten Kombination von Oxofarbstoffvorprodukten eine perfekte, langanhaltende und auch nach mehreren Haarwäschen noch reine Schwarznuance zu erzeugen.

Aufgabe der vorliegenden Erfindung ist es daher, ein Färbemittel für keratinhaltige Fasern, insbesondere menschliche Haare, zur Verfügung zu stellen, welches die Fasern auch ohne die Anwesenheit eines Oxidationsmittels wie beispielsweise Wasserstoffperoxid rein schwarz zu färben vermag und eine ausgesucht gute Waschechtheit besitzt. Darüber hinaus sollen die im Rahmen dieser Aufgabenstellung erzeugten Schwarznuancen auch nach mehreren Wäschen und auf unterschiedlich stark geschädigten Faserpartien keiner Farbverschiebung unterliegen und auch nach mindestens 6 Wäschen noch einen rein schwarzen Farbeindruck ohne Drift ins Braune, Blaue und Graue erzeugen.

Es wurde gefunden, dass sich spezielle halogensubstituierte Brenzcatechinaldehyde als Komponente (Oxo1) in Kombination mit speziellen CH-aciden Verbindungen als Komponente (Oxo2) auch in Abwesenheit von oxidierenden Agentien hervorragend zum Färben von keratinhaltigen Fasern eignen. Sie ergeben Ausfärbungen mit hervorragender Brillanz und Farbtiefe und erzielen insbesondere schwarze Farbnuancen. Es werden Ausfärbungen mit verbesserten Farbechtheitseigenschaften, insbesondere verbesserter Waschechtheit, erhalten. Die Egalisierung, d.h. die Gleichmäßigkeit der Färbung entlang der Faser, ist hervorragend und bleibt insbesondere auch nach mehrmaligem Waschen erhalten.

Ein erster Gegenstand der Erfindung ist daher ein Mittel zur Färbung keratinhaltiger Fasern, insbesondere menschlicher Haare, enthaltend in einem kosmetischen Träger

- mindestens eine reaktive Carbonylverbindung der Formel (I)

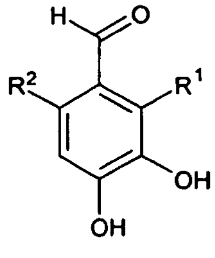

(I)

worin

genau einer der beiden Reste aus $R^1$ und $R^2$ für ein Wasserstoffatom steht und
genau einer der beiden Reste aus $R^1$ und $R^2$ für ein Halogenatom (insbesondere für Chlor, Fluor, Brom oder Iod) steht,

und

- mindestens eine CH-acide Verbindung der Formel (II)

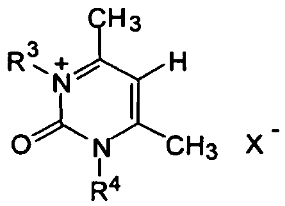

(II)

worin

- $R^3$ und $R^4$ stehen unabhängig voneinander für eine lineare oder cyclische ($C_1$ bis $C_6$)- Alkylgruppe, eine ($C_2$ bis $C_6$)- Alkenylgruppe, eine gegebenenfalls substituierte Arylgruppe, eine gegebenenfalls substituierte Heteroarylgruppe, eine Aryl- ($C_1$ bis $C_6$)- alkylgruppe, eine ($C_1$ bis $C_6$)- Hydroxyalkylgruppe, eine ($C_2$ bis $C_6$)- Polyhydroxyalkylgruppe, eine ($C_1$ bis $C_6$)- Alkoxy- ($C_1$ bis $C_6$)- alkylgruppe, eine Gruppe $R^IR^{II}N$- $(CH_2)_m$-, worin $R^I$ und $R^{II}$ stehen unabhängig voneinander für ein Wasserstoffatom, eine ($C_1$ bis $C_4$)- Alkylgruppe, eine ($C_1$ bis $C_4$)- Hydroxyalkylgruppe oder eine Aryl- ($C_1$ bis $C_4$)- alkylgruppe, wobei $R^I$ und $R^{II}$ gemeinsam mit dem Stickstoffatom einen 5-, 6- oder 7- gliedrigen Ring bilden können und m steht für eine Zahl 2, 3, 4, 5 oder 6,

- $X^-$ steht für ein physiologisch verträgliches Anion.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose verwendet werden.

Beispiele für ($C_1$ bis $C_6$)- Alkylreste sind die Gruppen- $CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$. Propyl, Ethyl und Methyl sind bevorzugte Alkylreste. Beispiele für bevorzugte ($C_2$ bis $C_6$)- Alkenylreste sind Vinyl, Allyl und Isobutenyl.
Weiterhin sind bevorzugte Beispiele für eine ($C_1$ bis $C_6$)- Monohydroxyalkylgruppe- $CH_2OH$, $-CH_2CH_2OH$, $-CH_2CH_2CH_2OH$, $-CHCH(OH)CH_3$, $-CH_2CH_2CH_2CH_2OH$, wobei die Gruppe- $CH_2CH_2OH$ bevorzugt ist.
Ein besonders bevorzugtes Beispiel einer ($C_2$ bis $C_6$)- Polyhydroxyalkylgruppe ist die 1, 2- Dihydroxyethylgruppe.
Erfindungsgemäße Beispiele für ($C_1$ bis $C_6$)- Alkoxyreste sind- $OCH_3$, $-OCH_2CH_3$, $-OCH_2CH_2CH_3$, $-OCH(CH_3)_2$, $-OCH_2CH_2CH_2CH_3$, $-OCH_2CH(CH_3)_2$, $-OCH(CH_3)CH_2CH_3$, $-OC(CH_3)_3$, insbesondere eine Methoxy- oder eine Ethoxygruppe.
Beispiele für ($C_1$ bis $C_6$)- Alkoxy- ($C_1$ bis $C_6$)- alkylgruppen sind die Gruppen- $CH_2CH_2OCH_3$, $-CH_2CH_2CH_2OCH_3$, $-CH_2CH_2OCH_2CH_3$, $-CH_2CH_2CH_2OCH_2CH_3$, $-CH_2CH_2OCH(CH_3)_2$, $-CH_2CH_2CH_2OCH(CH_3)_2$.
Beispiele für eine Arylgruppe sind die Phenyl- oder Naphthylgruppe.

Beispiele für Heteroarylgruppen sind Furyl, Thiophenyl, Pyrrolyl, Isoxazolyl, Isothiazolyl, Imidazolyl, Oxazolyl, Thiazolyl, Pyridyl, Pyridazyl, Pyrimidyl, Pyrazyl, 1, 2, 3- Triazinyl, 1, 2, 4- Triazinyl, 1, 3, 5- Triazinyl, Benzopyrrol, Benzofuryl, Benzothiophenyl, Benzimidazolyl, Benzoxazol, Indazolyl, Benzoisoxazolyl, Benzoisothiazolyl, Indolyl, Chinolyl, Isochinolyl, Cinnolyl, Phthalazyl, Chinazolyl, Chinoxalinyl, Acridinyl, Benzochinolyl, Benzoisochinolyl, Benzothiazolyl, Phenazinyl, Benzocinnolinyl, Benzochinazolyl, Benzochinoxalyl, Phenoxazinyl, Phenothiazinyl, Nephthyridyl, Phenanthrolinyl, Indolizinyl, Chinolizinyl und Carbolinyl. Die vorgenannten Aryl- bzw. Heteroarylgruppen können mit mindestens einer Gruppe ausgewählt aus einem Halogenatom, einer Nitrogruppe, einer Thiogruppe, einer Thio- ($C_1$ bis $C_6$)- alkylgruppe, einer Heteroarylgruppe, einer Arylgruppe, einer ($C_1$ bis $C_6$)- Alkylgruppe, einer ($C_1$ bis $C_6$)- Alkoxygruppe, einer Hydroxygruppe, einer ($C_2$ bis $C_6$)- Hydroxyalkylgruppe, einer ($C_2$ bis $C_6$)- Polyhydroxyalkylgruppe, einer ($C_1$ bis $C_6$)- Alkoxy- ($C_1$ bis $C_6$)- alkylgruppe, einer Aryl- ($C_1$ bis $C_6$)- alkylgruppe, einer Aminogruppe, einer Mono- (($C_1$ bis $C_6$)- alkyl) aminogruppe, einer Di- (($C_1$ bis $C_6$)- alkyl) aminogruppe, eine Dialkylaminoalkylgruppe- $(CH_2)_n$- NR'R", worin n eine ganze Zahl von 2 und 6 ist und R' und R" unabhängig voneinander eine lineare oder verzweigte Alkylgruppe

bedeutet, welche gegebenenfalls zusammen einen Ring bilden können, substituiert sein.
Die weiteren verwendeten Begriffe leiten sich erfindungsgemäß von den hier gegebenen Definitionen ab.

Besonders gute Ergebnisse wurden erhalten, wenn das erfindungsgemäße Mittel mindestens eine Verbindung gemäß Formel (I) enthält, in der $R^1$ für ein Halogenatom (insbesondere für Fluor, Chlor, Brom oder Iod, bevorzugt für Brom oder Chlor) und $R^2$ für ein Wasserstoffatom steht.

Im Rahmen einer bevorzugten Ausführungsform enthält das erfindungsgemäße Mittel mindestens eine Verbindung der Formel (I) ausgewählt aus mindestens einer Verbindung der Gruppe, die gebildet wird aus 2- Brom- 3, 4-dihydroxybenzaldehyd, 2- Chlor- 3, 4- dihydroxybenzaldehyd, 2- Fluor- 3, 4- dihydroxybenzaldehyd, 2- Iod- 3, 4- dihydroxybenzaldehyd, 2- Brom- 4, 5- dihydroxybenzaldehyd, 2- Chlor- 4, 5- dihydroxybenzaldehyd, 2- Fluor- 4, 5- dihydroxybenzaldehyd, 2- Iod- 4, 5- dihydroxybenzaldehyd.

Innerhalb dieser Gruppe zeichnen sich insbesondere die beiden Verbindungen 2- Brom- 3, 4- dihydroxybenzaldehyd sowie 2- Chlor- 3, 4- dihydroxybenzaldehyd durch herausragende anwendungstechnische Eigenschaften aus und sind daher einzeln oder gemeinsam explizit besonders bevorzugt in dem erfindungsgemäßen Mittel enthalten.

Das erfindungsgemäße Mittel enthält als weiteren zwingenden Bestandteil mindestens eine CH-acide Verbindung der Formel (II) *(vide supra)*.

Als CH-acide Verbindungen werden im Allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Prinzipiell sind der Auswahl der CH-aciden Verbindungen innerhalb der Maßgaben der Formel (II) keine Grenzen gesetzt. Nach der Kondensationsreaktion mit den erfindungsgemäßen Verbindungen der Formel (I) wird eine für das menschliche Auge sichtbar farbige Verbindung erhalten.

Es ist erfindungsgemäß bevorzugt als Verbindung der Formel (II) in dem erfindungsgemäßen Mittel mindestens eine Verbindung einzusetzen, bei der $R^3$ gemäß Formel (II) für eine Methylgruppe steht und $R^4$ gemäß Formel (II) für eine lineare oder cyclische ($C_1$ bis $C_6$)- Alkylgruppe, eine ($C_2$ bis $C_6$)- Alkenylgruppe, eine gegebenenfalls substituierte Arylgruppe, eine gegebenenfalls substituierte Heteroarylgruppe, eine Aryl- ($C_1$ bis $C_6$)- alkylgruppe, eine ($C_1$ bis $C_6$)-Hydroxyalkylgruppe oder eine ($C_2$ bis $C_6$)- Polyhydroxyalkylgruppe steht.

Vorzugsweise enthält das erfindungsgemäße Mittel als CH-acide Verbindung der Formel (II) mindestens eine CH-acide Verbindung gemäß Formel (II-a)

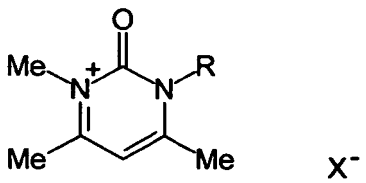

(II-a)

wobei

- R für eine Methylgruppe, eine Allylgruppe, eine Hydroxy- ($C_2$- bis $C_6$)- alkylgruppe oder eine gegebenenfalls substituierte Benzylgruppe steht,
- $X^-$ ein physiologisch verträgliches Anion bedeutet.

Beispiele für Hydroxy- ($C_2$- bis $C_6$)- alkylgruppen der Formel (II- a) sind 2- Hydroxyethyl, 2- Hydroxypropyl, 3-Hydroxypropyl, 4- Hydroxybutyl, und 6- Hydroxyhexyl. Bevorzugte Hydroxy- ($C_2$- bis $C_6$)- alkylgruppen gemäß Formel (II- a) sind 2- Hydroxyethyl, 2- Hydroxypropyl und 3- Hydroxypropyl.

R gemäß Formel (II-a) steht bevorzugt für eine Methylgruppe, eine Allylgruppe, eine 3-Hydroxypropylgruppe, eine 2-Hydroxypropylgruppe, eine 2-Hydroxyethylgruppe oder eine Benzylgruppe. Dabei ist es wiederum besonders bevorzugt, wenn das erfindungsgemäße Mittel als CH-acide Verbindung der Formel (II) mindestens eine Verbindung der Formel (II-a) enthält, in der R für eine Methylgruppe oder eine Allylgruppe steht.

Es ist bevorzugt, wenn $X^-$ gemäß Formel (II) bzw. (II- a) ausgewählt wird aus Halogenid, Benzolsulfonat, p-Toluolsulfonat, ($C_1$ bis $C_4$)- Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt steht $X^-$ für Chlorid, Bromid oder Hydrogensulfat.

Ein besonders bevorzugtes erfindungsgemäße Mittel enthält mindestens eine CH- acide Verbindung ausgewählt aus einer oder mehrerer Verbindungen der Gruppe von Salzen mit physiologisch verträglichem Gegenion $X^-$, die gebildet wird aus Salzen des

1, 2- Dihydro- 1, 3, 4, 6- tetramethyl- 2- oxo- pyrimidiniums,

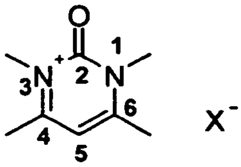

(II-1)

1- Allyl- 1, 2- dihydro- 3, 4, 6- trimethyl- 2- oxo- pyrimidiniums.

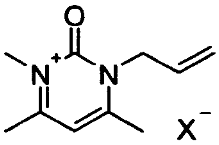

(II-2)

Die Nummerierung der ringbildenden Atome des Heterozyklus wird in obenstehender Formel (II-1) offenbart und in der Nomenklatur der Verbindungen der Formel (II) verwendet.

$X^-$ steht in den Formeln (II- 1) und (II- 2) sowie in obigen Listen bevorzugt für Halogenid, Benzolsulfonat, p-Toluolsulfonat, $C_1$- $C_4$- Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt werden die Anionen Chlorid, Bromid, Iodid, Hydrogensulfat oder p- Toluolsulfonat als $X^-$ eingesetzt.

Ganz besonders bevorzugt wird die CH- acide Verbindung der Formel (II) (bzw. (II- a) ) ausgewählt aus mindestens einer Verbindung der Gruppe bestehend aus 1- Allyl- 1, 2- dihydro- 3, 4, 6- trimethyl- 2- oxo- pyrimidiniumbromid und 1, 3, 4, 6- Tetramethyl- 2- oxo- pyrimidiniumhydrogensulfat.

Eine bevorzugte Ausführungsform der Erfindung stellt ein Mittel zur Färbung keratinhaltiger Fasern, insbesondere menschlicher Haare, dar, enthaltend in einem kosmetischen Träger

- als Komponente A mindestens eine Verbindung der Formel (I)

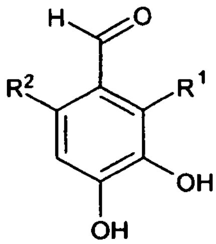

(I)

worin

$R^1$ für ein Halogenatom (insbesondere für Chlor, Fluor, Brom oder Iod) steht,
$R^2$ für ein Wasserstoffatom steht und

und

- als Komponente B mindestens eine CH-acide Verbindungen gemäß Formel (II-a)

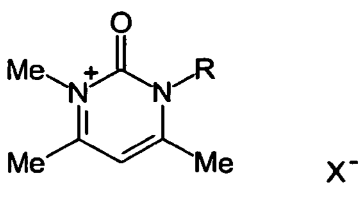

(II-a)

wobei

- R für eine Methylgruppe, eine Allylgruppe, eine Hydroxy- ($C_2$- bis $C_6$)- alkylgruppe oder eine gegebenenfalls substituierte Benzylgruppe steht,
- X- ein physiologisch verträgliches Anion bedeutet

Im Rahmen dieser Ausführungsform sind *mutatis mutandis* die bevorzugten Vertreter der Formel (I) bzw. die bevorzugten Vertreter der Formel (II) bzw. (II-a) wiederum besonders bevorzugt.

Dabei sind wiederum folgende Mittel M1 bis M8 der Tabelle 1 besonders bevorzugt, die folgende in einem kosmetischen Träger Verbindungen der Formeln (I) und (II) enthalten:

Tabelle 1:

| Mittel | Formel (I) | Formel (II) |
|---|---|---|
| M1 | Salz mit physiologisch verträglichem Gegenion $X^-$ des 1,2-Dihydro-1,3,4,6-tetramethyl-2-oxo-pyrimidiniums | 2-Brom-3,4-dihydroxybenzaldehyd |
| M2 | Salz mit physiologisch verträglichem Gegenion $X^-$ des 1,2-Dihydro-1,3,4,6-tetramethyl-2-oxo-pyrimidiniums | 2-Chlor-3,4-dihydroxybenzaldehyd |
| M3 | Salz mit physiologisch verträglichem Gegenion $X^-$ des 1-Allyl-1,2-Dihydro-3,4,9-trimethyl-2-oxo-pyrimidiniums | 2-Brom-3,4-dihydroxybenzaldehyd |
| M4 | Salz mit physiologisch verträglichem Gegenion $X^-$ des 1-Allyl-1,2-Dihydro-3,4,6-trimethyl-2-oxo-pyrimidiniums | 2-Chlor-3,4-dihydroxybenzaldehyd |
| M5 | 1,2-Dihydro-1,3,4,6-tetramethyl-2-oxo-pyrimidinium-hydrogensulfat | 2-Brom-3,4-dihydroxybenzaldehyd |
| M6 | 1-Allyl-1,2-Dihydro-3,4,6-tetramethyl-2-oxo-pyrimidiniumbromid | 2-Brom-3,4-dihydroxybenzaldehyd |
| M7 | 1,2-Dihydro-1,3,4,6-tetramethyl-2-oxo-pyrimidinium-hydrogensulfat | 2-Chlor-3,4-dihydroxybenzaldehyd |
| M8 | 1-Allyl-1,2-Dihydro-3,4,6-tetramethyl-2-oxo-pyrimidinium-bromid | 2-Chlor-3,4-dihydroxybenzaldehyd |

Es ist bevorzugt, wenn $X^-$ gemäß Tabelle 1 ausgewählt wird aus Halogenid, Benzolsulfonat, p- Toluolsulfonat, ($C_1$ bis $C_4$)- Alkansulfonat, Trifluormethansulfonat, Perchlorat, 0.5 Sulfat, Hydrogensulfat, Tetrafluoroborat, Hexafluorophosphat oder Tetrachlorozinkat. Besonders bevorzugt steht $X^-$ gemäß Tabelle 1 für Chlorid, Bromid oder Hydrogensulfat.

Die voranstehend genannten Verbindungen mit der Formel (I) werden vorzugsweise in einer Menge von 0,03 bis 65 mmol, insbesondere von 1 bis 40 mmol, bezogen auf 100 g des gesamten Färbemittels, verwendet. Entsprechende erfindungsgemäße Mittel, bei denen die Verbindungen der Formel (I) in einer Menge von 0,03 bis 65 mmol, insbesondere von 1 bis 40 mmol, bezogen auf 100 g des gesamten Färbemittels, enthalten sind, sind bevorzugt.

Die voranstehend genannten CH-aciden Verbindungen der Formel (II) (bzw. (II-a)) werden vorzugsweise in einer Menge von 0,03 bis 65 mmol, insbesondere von 1 bis 40 mmol, bezogen auf 100 g des gesamten Färbemittels, verwendet. Entsprechende erfindungsgemäße Mittel, bei denen die CH-aciden Verbindungen der Formel (II) (bzw. (II-a)) in einer Menge von 0,03 bis 65 mmol, insbesondere von 1 bis 40 mmol, bezogen auf 100 g des gesamten Färbemittels, enthalten sind, sind bevorzugt.

In einer weiteren Ausführungsform enthält das Färbemittel zusätzlich mindestens ein Reaktionsprodukt (im Folgenden als Reaktionsprodukt RP bezeichnet) aus einer Verbindung der Formel I und einer Verbindung der Komponente B als direktziehenden Farbstoff. Derartige Reaktionsprodukte RP können z. B. durch Erwärmen der beiden Reaktionspartner in wässrigem neutralen bis schwach alkalischen Milieu erhalten werden, wobei die Reaktionsprodukte RP entweder als Feststoff aus der Lösung ausfallen oder durch Eindampfen der Lösung daraus isoliert werden. Ferner besteht die Möglichkeit, die Reaktionsprodukte analog zu der Literaturvorschrift in H. Baumann et. al., Liebigs Ann. Chem. (1968), 717, 124-136 darzustellen.

Zur Synthese der Reaktionsprodukte RP können Molverhältnisse der Verbindung der Formel (II) zu der Verbindung gemäß Formel I von etwa 1:1 bis etwa 1:2 sinnvoll sein.

In einer weiteren Ausführungsform kann in dem erfindungsgemäßen Mittel zusätzlich mindestens eine Entwicklerkomponente und gegebenenfalls mindestens eine Kupplerkomponente als Oxidationsfarbstoffvorprodukte enthalten sein. Es ist jedoch erfindungsgemäß bevorzugt, die erfindungsgemäßen Mittel frei von Oxidationsfarbstoffvorprodukten - insbesondere frei von Oxidationsfarbstoffvorprodukten des Entwicklertyps - zu formulieren, insbesondere wenn ein Allergierisiko minimiert werden soll.

In einer weiteren Ausführungsform können die erfindungsgemäßen Färbemittel zur weiteren Modifizierung der Farbnuancen neben den erfindungsgemäß enthaltenen Verbindungen zusätzlich übliche direktziehende Farbstoffe enthalten, wie Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen bekannten Verbindungen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57: 1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1, 4- Diamino- 2- nitrobenzol, 2- Amino- 4- nitrophenol, 1, 4- Bis- (β-hydroxyethyl)- amino- 2- nitrobenzol, 3- Nitro- 4- (β- hydroxyethyl)- aminophenol, 2- (2'- Hydroxyethyl) amino- 4, 6-dinitrophenol, 1- (2'- Hydroxyethyl) amino- 4- methyl- 2- nitrobenzol, 1- Amino- 4- (2'- hydroxyethyl)- amino- 5- chlor- 2-nitrobenzol, 4- Amino- 3- nitrophenol, 1- (2'- Ureidoethyl) amino- 4- nitrobenzol, 4- Amino- 2- nitrodiphenylamin- 2'-carbonsäure, 6- Nitro- 1, 2, 3, 4- tetrahydrochinoxalin, 2- Hydroxy- 1, 4- naphthochinon, Pikraminsäure und deren Salze, 2- Amino- 6- chloro- 4- nitrophenol, 4- Ethylamino- 3- nitrobenzoesäure und 2- Chloro- 6- ethylamino- 1- hydroxy- 4-nitrobenzol. Bevorzugte erfindungsgemäße Mittel sind dadurch gekennzeichnet, daß sie zusätzlich mindestens einen direktziehenden Farbstoff, vorzugsweise in einer Menge von 0, 01 bis 20 Gew.- %, bezogen auf das gesamte Färbemittel, enthalten.

Ferner können die erfindungsgemäßen Mittel bevorzugt einen kationischen direktziehenden Farbstoff enthalten. Besonders bevorzugt sind dabei

(a) kationische Triphenylmethanfarbstoffe, wie beispielsweise Basic Blue 7, Basic Blue 26, Basic Violet 2 und Basic Violet 14,

(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel. Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten. Es ist nicht erforderlich, daß die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Zur Erlangung intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin- 2- carbonsäure, Piperidin- 3- carbonsäure, Piperidin- 4- carbonsäure, Pyridin, 2- Hydroxypyridin, 3- Hydroxypyridin, 4- Hydroxypyridin, Imidazol, 1- Methylimidazol, Arginin, Histidin, Pyrrolidin, Prolin, Pyrrolidon, Pyrrolidon- 5- carbonsäure, Pyrazol, 1, 2, 4- Triazol, Piperazidin, deren Derivate sowie deren physiologisch verträglichen Salzen.

Die voranstehend genannten Farbverstärker können in einer Menge von jeweils 0,03 bis 10 Gew.-%, insbesondere 0,5 bis 5 Gew.-%, jeweils bezogen auf 100 g des anwendungsbereiten Färbemittels, eingesetzt werden.

Die erfindungsgemäßen Mittel weisen optimalerweise einen pH-Wert von pH 4 bis 12, bevorzugt von pH 5 bis 10, auf. Dabei eignet sich insbesondere bevorzugt ein pH-Wert von größer pH 7, besonders bevorzugt von pH 7,5 bis pH 11 in hervorragendem Maße. Ganz besonders bevorzugt besitzen die erfindungsgemäßen Mittel einen pH-Wert von pH 8 bis 10.

Die erfindungsgemäßen Färbemittel ergeben bereits in wässrigem Medium bei physiologisch verträglichen Temperaturen von unter 45°C intensive Färbungen. Sie eignen sich deshalb besonders zum Färben von menschlichen Haaren.

Als kosmetischer Träger wird erfindungsgemäß insbesondere ein ansonsten üblicher Träger von Mitteln zur Färbung menschlicher Haare eingesetzt. Die erfindungsgemäßen Färbemittel können dabei, abgesehen von den erfindungsgemäßen Komponenten, entsprechend bekannter Färbemittel zusammengesetzt sein bzw. die für diese üblichen Inhaltsstoffe enthalten. Beispiele weiterer geeigneter und erfindungsgemäß bevorzugter Inhaltsstoffe sind nachstehend angegeben.

Die erfindungsgemäßen Mittel enthalten die Verbindungen der Formel (I) und die CH-aciden Verbindungen der Formel (II) bevorzugt in einem kosmetischen sowie wässrigen oder alkoholischen oder wässrig-alkoholischen Träger.

Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Es ist aber auch denkbar, die Oxofarbstoffvorprodukte in eine pulverförmige oder auch tabletten-förmige Formulierung zu integrieren.

Unter wässrig- alkoholischen Lösungen sind im Sinne der vorliegenden Erfindung wässrige Lösungen enthaltend 3 bis 70 Gew.- % eines $C_1$- $C_4$- Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1, 2- Propylenglykol, enthalten. Bevorzugt sind dabei alle wasserlöslichen organischen Lösemittel.

In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen, so daß erfindungsgemäße Mittel, die zusätzlich anionische, zwitterionische oder nichtionische Tenside enthalten, bevorzugt sind.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanol-ammoniumsalze mit 2 oder 3 C-Atomen in der Alkanolgruppe,

- lineare Fettsäuren mit 10 bis 22 C-Atomen (Seifen),
- Ethercarbonsäuren der Formel R- O- $(CH_2- CH_2O)_x$- $CH_2$- COOH, in der R eine lineare Alkylgruppe mit 10 bis 22 C- Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R- O $(CH_2- CH_2O)_x$- $SO_3H$, in der R eine bevorzugt lineare Alkylgruppe mit 10 bis 18 C- Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C- Atomen in der Alkylgruppe und bis zu 12 Glykolethergruppen im Molekül sowie insbesondere Salze von gesättigten und insbesondere ungesättigten $C_8$- $C_{22}$- Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine- $COO^{(-)}$- oder- $SO_3^{(-)}$- Gruppe tragen. Besonders geeignete zwitterionische Tenside sind die sogenannten Betaine wie die N- Alkyl- N, N- dimethylammonium- glycinate, beispielsweise das Kokosalkyl- dimethylammoniumglycinat, N- Acyl- aminopropyl- N, N- dimethylammoniumglycinate, beispielsweise das Kokosacylaminopropyl- dimethylammoniumglycinat, und 2- Alkyl- 3- carboxymethyl- 3- hydroxyethyl-imidazoline mit jeweils 8 bis 18 C- Atomen in der Alkyl- oder Acylgruppe sowie das Kokosacylaminoethylhydroxyethylcarboxymethylglycinat. Ein bevorzugtes zwitterionisches Tensid ist das unter der CTFA- Bezeichnung Cocamidopropyl Betaine bekannte Fettsäureamid- Derivat.

Unter ampholytischen Tensiden werden solche oberflächenaktiven Verbindungen verstanden, die außer einer $C_{8-18}$- Alkyl- oder- Acylgruppe im Molekül mindestens eine freie Aminogruppe und mindestens eine- COOH- oder- $SO_3H$-Gruppe enthalten und zur Ausbildung innerer Salze befähigt sind. Beispiele für geeignete ampholytische Tenside sind N- Alkylglycine, N- Alkylpropionsäuren, N- Alkylaminobuttersäuren, N- Alkyliminodipropionsäuren, N- Hydroxyethyl- N-alkylamidopropylglycine, N- Alkyltaurine, N- Alkylsarcosine, 2- Alkylaminopropionsäuren und Alkylaminoessigsäuren mit jeweils etwa 8 bis 18 C- Atomen in der Alkylgruppe. Besonders bevorzugte ampholytische Tenside sind das N-Kokosalkylaminopropionat, das Kokosacylaminoethylaminopropionat und das $C_{12-18}$- Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12-22}$- Fettsäuremono- und- diester von Anlagerungsprodukten von 1 bis 30 Mol Ethylenoxid an Glycerin,
- $C_{8-22}$- Alkylmono- und- oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise

- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid- Polymere, Acrylamid- Dimethyldiallylammoniumchlorid- Copolymere, mit Diethylsulfat quaternierte Dimethylaminoethylmethacrylat- Vinylpyrrolidon- Copolymere, Vinylpyrrolidon- Imidazoliniummethochlorid- Copolymere und quatemierter Polyvinylalkohol,
- zwitterionische und amphotere Polymere wie beispielsweise Acrylamidopropyl- trimethyl- ammoniumchlorid/ Acrylat-Copolymere und Octylacrylamid/ Methylmethacrylat/ tert.- Butylaminoethylmethacrylat/ 2- Hydroxypropylmethacrylat- Copolymere,
- anionische Polymere wie beispielsweise Polyacrylsäuren, vernetzte Polyacrylsäuren, Vinylacetat/ Crotonsäure-Copolymere, Vinylpyrrolidon/ Vinylacrylat- Copolymere, Vinylacetat/ Butylmaleat/ Isobornylacrylat- Copolymere, Methylvinylether/ Maleinsäureanhydrid- Copolymere und Acrylsäure/ Ethylacrylat/N- tert.- Butylacrylamid- Terpolymere,

- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Antischuppenwirkstoffe wie Piroctone Olamine und Zink Omadine,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Trübungsmittel wie Latex,
- Perlglanzmittel wie Ethylenglykolmono- und -distearat,
- Treibmittel wie Propan- Butan- Gemische, $N_2O$, Dimethylether, $CO_2$ und Luft sowie
- Antioxidantien.

Die Bestandteile des kosmetischen Trägers werden zur Herstellung der erfindungsgemäßen Mittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Mittels eingesetzt.

Das erfindungsgemäße Mittel wird bevorzugt unmittelbar vor der Anwendung durch vermischen der mindestens zweier Komponenten- i.e. Komponente (Oxo1) und Komponente (Oxo2)- erhalten. Daher liegt das erfindungsgemäße Mittel bevorzugt vor der Anwendung als Kit (Verpackungseinheit) vor. Dieses Kit (Verpackungseinheit) umfasst als bevorzugte Konfektionierungsart der erfindungsgemäßen Mittel mindestens ein Mittel A1 und mindestens ein Mittel A2, mit den Maßgaben, dass

- das Mittel A1 in einem kosmetischen Träger mindestens eine CH-acide Verbindung der zuvor definierten Formel (II) enthält und
- das Mittel A2 in einem kosmetischen Träger mindestens eine Verbindung der zuvor definierten Formel (I) enthält und
- die Mittel A1 und A2 jeweils getrennt konfektioniert vorliegen.

Werden die Mittel A1 und A2 vermischt, wird das erfindungsgemäße Mittel des ersten Erfindungsgegenstandes erhalten.

Getrennt konfektioniert bedeutet im Sinne der Erfindung, dass die betroffenen Mittel jeweils in einem eigenen Container konfektioniert sind. Als Container können unterschiedliche Verpackungsarten dienen, wie beispielsweise Folien, Beutel, Flaschen, Dosen, Aerosolbehältnisse. Ebenso gilt eine Kammer eines Mehrkammerbehältnisses als Container.

Im Rahmen dieser Ausführungsform sind die Mittel A1 somit frei von reaktiven Carbonylverbindungen und die Mittel A2 frei von CH-aciden Verbindungen.

Das anwendungsbereite Färbemittel - beziehungsweise das Mittel A1 - enthält mindestens eine CH-acide Verbindung der besagten Formel (II) (*vide supra*).

Das anwendungsbereite Färbemittel - beziehungsweise das Mittel A2 - enthält mindestens eine Verbindung der besagten Formel (I) *(vide supra*).

Hierbei sind *mutatis mutandis* die bevorzugten Ausführungsformen des erfindungsgemäßen Mittels ebenfalls bevorzugt.

Die Mittel A1 und A2 können im Rahmen einer Ausführungsform in dem Kit pulverförmig, granuliert oder als Formkörper vorliegen.

Ein pulverförmiges Mittel A1 bzw. A2 besitzt eine bevorzugte mittlere Teilchengröße von 0,0001 bis 50 $\mu$m, insbesondere von 0,05 bis 30 $\mu$m.

Als Granulate werden erfindungsgemäß körnige Partikel verstanden. Diese körnigen Partikel sind fließfähig.

Granulate können durch Feuchtgranulierung, durch Trockengranulierung bzw. Kompaktierung und durch

Schmelzerstarrungsgranulierung hergestellt werden. Die gebräuchlichste Granuliertechnik ist die Feuchtgranulierung, da diese Technik den wenigsten Einschränkungen unterworfen ist und am sichersten zu Granulaten mit günstigen Eigenschaften führt. Die Feuchtgranulierung erfolgt durch Befeuchtung der Pulvermischungen mit Lösungsmitteln und/ oder Lösungsmittelgemischen und/ oder Lösungen von Bindemitteln und/ oder Lösungen von Klebstoffen und wird vorzugsweise in Mischern, Wirbelbetten oder Sprühtürmen durchgeführt, wobei besagte Mischer beispielsweise mit Rühr- und Knetwerkzeugen ausgestattet sein können. Für die Granulation sind jedoch auch Kombinationen von Wirbelbett (en) und Mischer (n), bzw. Kombinationen verschiedener Mischer einsetzbar. Die Granulation erfolgt unter Einwirkung niedriger bis hoher Scherkräfte.

Wenn die Mittel A1 bzw. A2 als Formkörper vorliegt, dann können diese erfindungsgemäßen Formkörper jedwede geometrische Form aufweisen, wie beispielsweise konkave, konvexe, bikonkave, bikonvexe, kubische, tetragonale, orthorhombische, zylindrische, sphärische, zylindersegmentartige, scheibenförmige, tetrahedrale, dodecahedrale, octahedrale, konische, pyramidale, ellipsoide, fünf-, sieben- und achteckig-prismatische sowie rhomboedrische Formen. Auch völlig irreguläre Grundflächen wie Pfeil- oder Tierformen, Bäume, Wolken usw. können realisiert werden. Die Ausbildung als Tafel, die Stab- bzw. Barrenform, Würfel, Quader und entsprechende Raumelemente mit ebenen Seitenflächen sowie insbesondere zylinderförmige Ausgestaltungen mit kreisförmigem oder ovalem Querschnitt und Formkörper mit sphärischer Geometrie sind erfindungsgemäß bevorzugt. Besonders bevorzugt sind Formkörper in Gestalt sphärischer Geometrie.

Die zylinderförmige Ausgestaltung erfaßt dabei die Darbietungsform von der Tablette bis zu kompakten Zylinderstücken mit einem Verhältnis von Höhe zu Durchmesser größer 1. Weist der Basisformkörper Ecken und Kanten auf, so sind diese vorzugsweise abgerundet. Als zusätzliche optische Differenzierung ist eine Ausführungsform mit abgerundeten Ecken und abgeschrägten ("angefasten") Kanten bevorzugt.

Die sphärische Ausgestaltung umfaßt neben einer kugelförmigen Gestalt auch einen Hybrid aus Kugel- und Zylinderform, wobei jede Grundfläche des Zylinders mit je einer Halbkugel überkappt ist. Die Halbkugeln haben bevorzugt einen Radius von ca. 4 mm und der gesamte Formkörper dieser Ausgestaltung eine Länge von 12 -14 mm.

Ein erfindungsgemäßer Formkörper mit sphärischer Ausgestaltung kann nach den bekannten Verfahren hergestellt werden. Es ist dabei möglich, die Formkörper durch Extrusion eines Vorgemisches mit nachfolgender Formgebung zu produzieren, wie es zum Beispiel in der WO-A-91/02047 näher ausgeführt ist, auf die im Rahmen dieser Anmeldung ausdrücklich Bezug genommen wird.

In einer weiteren bevorzugten Ausführungsform werden daher nahezu kugelförmige Formkörper, insbesondere durch Extrusion und nachfolgender Verrundung zur Formgebung, hergestellt.

In einer weiteren Ausführungsform können die portionierten Preßlinge dabei jeweils als voneinander getrennte Einzelelemente ausgebildet sein, die der vorbestimmten Dosiermenge der CH-aciden Verbindungen der Formel (II) beziehungsweise der Verbindungen der Formel (I) entspricht. Ebenso ist es aber möglich, Preßlinge auszubilden, die eine Mehrzahl solcher Masseneinheiten in einem Preßling verbinden, wobei insbesondere durch vorgegebene Sollbruchstellen die leichte Abtrennbarkeit portionierter kleinerer Einheiten vorgesehen ist. Die Ausbildung der portionierten Preßlinge als Tabletten in Zylinder- oder Quaderform kann zweckmäßig sein, wobei ein Durchmesser/Höhe-Verhältnis im Bereich von etwa 0,5 : 2 bis 2 : 0,5 bevorzugt ist. Handelsübliche Hydraulikpressen, Exzenterpressen oder Rundläuferpressen sind geeignete Vorrichtungen insbesondere zur Herstellung derartiger Preßlinge.

Eine weitere mögliche Raumform der erfindungsgemäßen Formkörper weist eine rechteckige Grundfläche auf, wobei die Höhe der Formkörper kleiner ist als die kleinere Rechteckseite der Grundfläche. Abgerundete Ecken sind bei dieser Angebotsform bevorzugt.

Ein weiterer Formkörper, der hergestellt werden kann, hat eine platten- oder tafelartige Struktur mit abwechselnd dicken langen und dünnen kurzen Segmenten, so daß einzelne Segmente von diesem "Riegel" an den Sollbruchstellen, die die kurzen dünnen Segmente darstellen, abgebrochen und derartig portioniert zum Einsatz kommen können. Dieses Prinzip des "riegelförmigen" Formkörpers kann auch in anderen geometrischen Formen, beispielsweise senkrecht stehenden Dreiecken, die lediglich an einer ihrer Seiten längsseits miteinander verbunden sind, verwirklicht werden.

Enthalten die erfindungsgemäßen Formkörper neben den erfindungsgemäßen Komponenten zusätzlich mindestens eine weitere Komponente, kann es in einer weiteren Ausführungsform vorteilhaft sein, die verschiedenen Komponenten nicht ausschließlich zu einer einheitlichen

Tablette zu verpressen. Bei der Tablettierung werden in dieser Ausführungsform Formkörper erhalten, die mehrere Schichten, also mindestens zwei Schichten, aufweisen. Dabei ist es auch möglich, daß diese verschiedenen Schichten unterschiedliche Lösegeschwindigkeiten aufweisen. Hieraus können vorteilhafte anwendungstechnische Eigenschaften der Formkörper resultieren. Falls beispielsweise Komponenten in den Formkörpern enthalten sind, die sich wechselseitig negativ beeinflussen, so ist es möglich, die eine Komponente in der schneller löslichen Schicht zu integrieren und die andere Komponente in eine langsamer lösliche Schicht einzuarbeiten, so daß die Komponenten nicht bereits während des Lösevorgangs miteinander reagieren.

Der Schichtaufbau der Formkörper kann dabei sowohl stapelartig erfolgen, wobei ein Lösungsvorgang der inneren Schicht(en) an den Kanten des Formkörpers bereits dann erfolgt, wenn die äußeren Schichten noch nicht

vollständig gelöst sind. Bei der stapelförmigen Anordnung kann die Stapelachse beliebig zur Tablettenachse angeordnet sein. Die Stapelachse kann also beispielsweise bei einer zylinderförmigen Tablette parallel oder senkrecht zur Höhe des Zylinders liegen.

Es kann aber auch gemäß einer weiteren Ausführungsform bevorzugt sein, wenn eine vollständige Umhüllung der inneren Schicht(en) durch die jeweils weiter außen liegende(n) Schicht(en) erreicht wird, was zu einer Verhinderung der frühzeitigen Lösung von Bestandteilen der inneren Schicht(en) führt. Bevorzugt sind Formkörper, bei denen die Schichten mit den verschiedenen Wirkstoffen sich umhüllen. Beispielsweise sei eine Schicht (A) vollständig von der Schicht (B) und diese wiederum vollständig von der Schicht (C) umhüllt. Ebenso können Formkörper bevorzugt sein, bei denen z.B. die Schicht (C) vollständig von der Schicht (B) und diese wiederum vollständig von der Schicht (A) umhüllt ist.

Ähnliche Effekte lassen sich auch durch Beschichtung ("coating") einzelner Bestandteile der zu verpressenden Zusammensetzung oder des gesamten Formkörpers erreichen. Hierzu können die zu beschichtenden Körper beispielsweise mit wäßrigen Lösungen oder Emulsionen bedüst werden, oder aber über das Verfahren der Schmelzbeschichtung einen Überzug erhalten. Als erfindungsgemäß geeignet zeigt sich beispielsweise die Verwendung einer Beschichtung aus Hydroxypropy-Methylcellulose, Cellulose, PEG-Stearaten und Farbpigmenten.

Die Herstellung der erfindungsgemäßen Formkörper erfolgt zunächst durch das trockene Vermischen der Bestandteile, die ganz oder teilweise vorgranuliert sein können, und anschließendes Informbringen, insbesondere Verpressen zu Tabletten, wobei auf bekannte Verfahren zurückgegriffen werden kann. Zur Herstellung der erfindungsgemäßen Formkörper wird das Vorgemisch in einer sogenannten Matrize zwischen zwei Stempeln zu einem festen Komprimat verdichtet. Dieser Vorgang, der im folgenden kurz als Tablettierung bezeichnet wird, gliedert sich in vier Abschnitte: Dosierung, Verdichtung (elastische Verformung), plastische Verformung und Ausstoßen.

Die Tablettierung erfolgt in handelsüblichen Tablettenpressen, die prinzipiell mit Einfach- oder Zweifachstempeln ausgerüstet sein können. Im Rahmen der vorliegenden Erfindung geeignete Tablettiermaschinen sind beispielsweise erhältlich bei den Firmen Apparatebau Holzwarth GbR, Asperg, Wilhelm Fette GmbH, Schwarzenbek, Fann Instruments Company, Houston, Texas (USA), Hofer GmbH, Weil, Horn & Noack - Pharmatechnik GmbH, Worms, IMA Verpackungssysteme GmbH Viersen, KILIAN, Köln, KOMAGE, Kell am See, KORSCH Pressen AG, Berlin, sowie Romaco GmbH, Worms. Weitere Anbieter sind beispielsweise Dr. Herbert Pete, Wien (AT), Mapag Maschinenbau AG, Bem (CH), BWI Manesty, Liverpool (GB), I. Holand Ltd., Nottingham (GB), Courtoy N.V., Halle (BE/LU) sowie Mediopharm Kamnik (SI). Besonders geeignet ist beispielsweise die Hydraulische Doppeldruckpresse HPF 630 der Firma LAEIS, D. Tablettierwerkzeuge sind beispielsweise von den Firmen Adams Tablettierwerkzeuge, Dresden, Wilhelm Fett GmbH, Schwarzenbek, Klaus Hammer, Solingen, Herber % Söhne GmbH, Hamburg, Hofer GmbH, Weil, Horn & Noack, Pharmatechnik GmbH, Worms, Ritter Pharamatechnik GmbH, Hamburg, Romaco, GmbH, Worms und Notter Werkzeugbau, Tamm erhältlich. Weitere Anbieter sind z.B. die Senss AG, Reinach (CH) und die Medicopharm, Kamnik (SI).

Die als Feststoff konfektionierten Mittel A1 und/oder A2 enthalten im Rahmen einer bevorzugten Ausführungsform mindestens ein Alkalisierungsmittel.

Diese Alkalisierungsmittel werden wiederum bevorzugt aus mindestens einem Alkalisierungsmittel ausgewählt aus der Gruppe, die gebildet wird, basischen Aminosäuren, Alkalihydroxiden, Alkanolaminen, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L- Arginin, D- Arginin, D, L- Arginin, L- Histidin, D- Histidin, D, L- Histidin, L- Lysin, D- Lysin, D, L- Lysin, besonders bevorzugt L- Arginin, D- Arginin, D, L- Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$- $C_6$- Alkylgrundkörper, der mindestens eine Hydroxylgruppe trägt. Besonders bevorzugte Alkanolamine werden aus der Gruppe ausgewählt, die gebildet wird, aus 2- Aminoethan- 1- ol (Monoethanolamin), 3- Aminopropan- 1- ol, 4- Aminobutan- 1- ol, 5- Aminopentan- 1- ol, 1- Aminopropan- 2- ol, 1- Aminobutan- 2- ol, 1- Aminopentan- 2- ol, 1- Aminopentan- 3- ol, 1- Aminopentan- 4- ol, 3- Amino- 2- methylpropan- 1- ol, 1- Amino- 2- methylpropan- 2- ol, 3- Aminopropan- 1, 2- diol, 2- Amino- 2- methylpropan- 1, 3- diol. Erfindungsgemäß ganz besonders bevorzugte Alkanolamine werden ausgewählt aus der Gruppe 2- Aminoethan- 1- ol, 2- Amino- 2- methylpropan- 1- ol und 2- Amino- 2- methyl- propan- 1, 3- diol.

Besonders bevorzugt wird das Alkalisierungsmittel ausgewählt aus mindestens einer Verbindung aus der Gruppe, die gebildet wird aus 2- Aminoethanol, 2- Amino- 2- methylpropan- 1- ol, 2- Amino- 2- methyl- propan- 1, 3- diol, Kaliumhydroxid, Natriumhydroxid, L- Arginin, D- Arginin, DL- Arginin, N- Methylglucamin, Morpholin, Imidazol und Harnstoff.

Im Rahmen der Ausführungsform der als Feststoff vorliegenden Mittel A1 und A2 sind die als Feststoff vorliegenden Alkalisierungmittel besonders bevorzugt.

Die als Pulver, Granulat oder Formkörper vorliegenden Mittel A1 und A2 werden dann gemeinsam in einem flüssigen kosmetischen Mittel A3 oder in Wasser unter Erhalt des anwendungsbereiten Färbemittels gemischt. Das flüssige kosmetische Mittel A3 kann im Rahmen dieser Ausführungsform ebenso vom erfindungsgemäßen Kit umfasst sein.

Als flüssige kosmetische Träger für das Mittel A3 eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Die flüssigen kosmetischen Träger können insbesondere wässrig oder wässrigalkoholisch sein.

Unter flüssig wird erfindungsgemäß verstanden, wenn der kosmetische Träger bei 25°C bei einem Druck von 1 atm einen flüssigen Aggregatzustand besitzt.

Ein wässriger kosmetischer Träger enthält mindestens 50 Gew.-% Wasser.

Unter wässrig-alkoholischen kosmetischen Trägem sind im Sinne der vorliegenden Erfindung wässrige Lösungen enthaltend 3 bis 70 Gew.-% eines Alkohols.

Es ist erfindungsgemäß bevorzugt, wenn das Mittel A3 zusätzlich mindestens ein organisches Lösemittel enthält. Dieses organische Lösemittel wird wiederum bevorzugt ausgewählt aus der Gruppe, die gebildet wird, aus

- ($C_1$ bis $C_4$)- Monohydroxyalkoholen,
- ($C_3$ bis $C_6$)- Dihydroxyalkoholen,
- ($C_3$ bis $C_6$)- Trihydroxyalkoholen,
- zyklischen, organischen Carbonaten,
- Verbindungen der Formel H- (O- $CH_2CH_2)_n$- OR mit R = Wasserstoffatom, Methylgruppe und n = 1, 2, 3 oder 4

Als bevorzugte ($C_1$ bis $C_4$)- Monohydroxyalkohole gelten Ethanol und Isopropanol.

Als bevorzugter ($C_3$ bis $C_6$)- Dihydroxyalkohol gilt 1, 2- Propandiol.

Als bevorzugter ($C_3$ bis $C_6$)- Trihydroxyalkohol gilt Glyzerin.

Als zyklisches, organisches Carbonat eignet sich erfindungsgemäß bevorzugt mindestens ein zyklischer Kohlensäureester. Diese zyklischen Ester der Kohlensäure leiten sich vom 1, 3- Dioxolan- 2- on ab und lassen sich durch folgende Grundstruktur der Formel (III- 1) beschreiben:

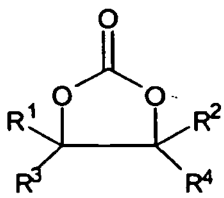
(III-1)

worin die Reste $R^1$, $R^2$, $R^3$ und $R^4$ unabhängig voneinander für ein Wasserstoffatom oder organische Reste, insbesondere Alkyl-, Alkenyl- oder Alkylaryl, stehen, die zusätzlich mit weiteren Gruppen, insbesondere Hydroxygruppen, substituiert sein können.

Im Grundkörper, dem 1, 3- Dioxolan- 2- on, stehen die Reste $R^1$, $R^2$, $R^3$ und $R^4$ der Formel (III- 1) jeweils für ein Wasserstoffatom. Weiterhin bevorzugt geeignete cyclische Kohlensäureester betreffen Derivate dieses Grundkörpers, wobei mindestens einer der Reste $R^1$, $R^2$, $R^3$ und $R^4$ der Formel (III- 1) von einem Wasserstoffatom verschieden ist. Hierbei sind der strukturellen Vielfalt keine Grenzen gesetzt, so daß sich mono-, di-, tri- und tetra- substituierte 1, 3- Dioxolan- 2- one der Formel (III- 1) zum Einsatz im Rahmen der vorliegenden Erfindung eignen.

Besonders bevorzugt sind neben dem unsubstituierten 1, 3- Dioxolan- 2- on insbesondere die in 4- Stellung monosubstituierten Derivate der nachstehenden Formel (III- 2)

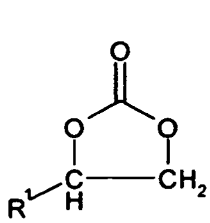
(III-2)

in der $R^1$ für einen substituierten oder unsubstituierten Alkyl-, Alkenyl- oder Alkylarylrest steht. Bevorzugte Reste $R^1$

gemäß Formel (III- 2) sind Methyl-, Ethyl-, n- Propyl-, iso- Propyl- sowie Hydroxymethyl-, 1- Hydroxyethyl- und 2- Hydroxyethyl- Reste.

Besonders bevorzugte erfindungsgemäße Mittel sind folglich dadurch gekennzeichnet, daß sie als 1, 3- Dioxolan- 2- on- Derivat mindestens eine Verbindung der obigen Formel (III- 2) enthalten, bei der $R^1$ für einen substituierten oder unsubstituierten Alkyl-, Alkenyl- oder Alkylarylrest steht, wobei in weiter bevorzugten erfindungsgemäßen Mitteln der Rest $R^1$ in Formel (III- 2) ausgewählt ist aus Methyl-, Ethyl-, n- Propyl-, iso- Propyl- sowie Hydroxymethyl-, 1- Hydroxyethyl- und 2- Hydroxyethyl- Resten.

Besonders bevorzugte 1, 3- Dioxolan- 2- one der Formel (III- 1) stammen aus der Gruppe Ethylencarbonat ($R^1$, $R^2$, $R^3$ und $R^4$ = H), Propylencarbonat (R' = $CH_3$ sowie $R^2$, $R^3$ und $R^4$ = H) und Glycerincarbonat (R' = $CH_2OH$ sowie $R^2$, $R^3$ und $R^4$ = H) . Ganz besonders bevorzugt eignet sich Propylencarbonat.

Bevorzugte Verbindungen der Formel H- (O- $CH_2CH_2)_n$- OR mit R = Wasserstoffatom, Methylgruppe und n = 1, 2, 3 oder 4 werden ausgewählt aus Ethylenglycol, Diethylenglycol, Diethylenglycolmonomethylether.

Besonders bevorzugt ist ein Gemisch aus

Ethylenglycol und Isopropanol,

oder aus

Isopropanol, Propylencarbonat und Diethylenglycol

als organische Lösemittel zu verwenden.

Die organischen Lösemittel werden im Mittel A3 bevorzugt in einer Menge von 0,5 bis 20 Gew.-%, insbesondere von 2 bis 10 Gew.-%, ganz besonders von 3 bis 6 Gew.-%, jeweils bezogen auf das Gewicht des Mittels A3, eingesetzt.

Im Rahmen einer besonders bevorzugten Ausführungsform, liegt das Kit umfassend folgende Komponenten vor:

- mindestens ein Mittel A1 mit einem pH-Wert von 0,5 bis 2,5 enthaltend in einem flüssigen kosmetischen Träger mindestens eine CH-acide Verbindung der zuvor definierten Formel (II) und
- mindestens ein Mittel A2 mit einem pH-Wert von 2 bis 5, enthaltend in einem flüssigen kosmetischen Träger mindestens eine Verbindung der zuvor definierten Formel (I),
- die Mittel A1 und A2 liegen jeweils getrennt konfektioniert vor.

Besonders lagerstabile Formulierungen können erhalten werden, wenn das Mittel A1 einen pH-Wert von 1 bis 2 und insbesondere bevorzugt einen pH-Wert von 1,1 bis 1,9 aufweist, daher ist dieser pH-Bereich bevorzugt.

Desweiteren ist es bevorzugt, wenn das Mittel A2 einen pH-Wert von 3 bis 5, insbesondere von 3,2 bis 4,5, aufweist.

Bei den pH-Werten im Sinne der vorliegenden Erfindung handelt es sich um pH-Werte, die bei einer Temperatur von 22 °C gemessen wurden.

Die Einstellung des pH-Wertes in den Mitteln A1 und/oder A2 kann mit Hilfe einer organischen oder anorganischen Säure wie beispielsweise Salzsäure, Schwefelsäure, Bromwasserstoffsäure, Phosphorsäure, Essigsäure, Weinsäure, Zitronensäure, Milchsäure, Äpfelsäure oder Glykolsäure erfolgen.

In diesem Zusammenhang ist die Einstellung der erfindungsgemäßen pH-Werte mit Hilfe von Salzsäure, Weinsäure, Zitronensäure, Äpfelsäure oder Milchsäure besonders bevorzugt.

Für die Anwendung des erfindungsgemäßen Kits können die Mittel A1 und A2 kurz vor dem Aufbringen auf die Haarfaser innig miteinander vermischt werden. Zur weiteren Verbesserung des Färbeergebnisses kann es jedoch vorteilhaft sein, die Färbung selbst in einem alkalischen pH-Bereich durchzuführen.

Daher kann im Rahmen einer besonderen Ausführungsform das erfindungsgemäße Kit zusätzlich ein drittes, zuvor beschriebenes flüssiges kosmetisches Mittel A3 (*vide supra*) umfassen, welches in einem kosmetischen Träger mindestens ein Alkalisierungsmittel enthält und einen pH-Wert größer als 7 aufweist. Diese Ausführungsform des Kits ist für die Ausführungsform von A1 und A2 als Flüssigkeiten mit speziellem sauren pH-Wert besonders bevorzugt.

Erfindungsgemäß sind somit solche Kits bevorzugt, in denen die Mittel A1, A2 und A3 so ausgestaltet sind, dass das anwendungsbereite Mittel einen pH-Wert größer 7 aufweist.

Die flüssigen kosmetischen Mittel A3 enthalten im Rahmen dieser Ausgestaltung des Kits bevorzugt zusätzlich mindestens ein Alkalisierungsmittel.

Die im flüssigen kosmetischen Mittel A3 enthaltenen Alkalisierungsmittel werden bevorzugt aus mindestens einem Alkalisierungsmittel ausgewählt aus der Gruppe, die gebildet wird, aus Ammoniak, basischen Aminosäuren, Alkalihydroxiden, Alkanolaminen, Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L- Arginin, D- Arginin, D, L- Arginin, L- Histidin, D- Histidin, D, L- Histidin, L- Lysin, D- Lysin, D, L- Lysin, besonders bevorzugt L- Arginin, D- Arginin, D, L- Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem $C_2$- $C_6$- Alkylgrundkörper, der mindestens eine Hydroxylgruppe trägt. Besonders bevorzugte Alkanolamine werden aus der Gruppe ausgewählt, die gebildet wird, aus 2- Aminoethan- 1- ol (Monoethanolamin), 3-Aminopropan- 1- ol, 4- Aminobutan- 1- ol, 5- Aminopentan- 1- ol, 1- Aminopropan- 2- ol, 1- Aminobutan- 2- ol, 1-Aminopentan- 2- ol, 1- Aminopentan- 3- ol, 1- Aminopentan- 4- ol, 3- Amino- 2- methylpropan- 1- ol, 1- Amino- 2-methylpropan- 2- ol, 3- Aminopropan- 1, 2- diol, 2- Amino- 2- methylpropan- 1, 3- diol. Erfindungsgemäß ganz besonders bevorzugte Alkanolamine werden ausgewählt aus der Gruppe 2- Aminoethan- 1- ol, 2- Amino- 2- methylpropan- 1- ol und 2- Amino- 2- methyl- propan- 1, 3- diol.

Besonders bevorzugt wird das Alkalisierungsmittel ausgewählt aus mindestens einer Verbindung aus der Gruppe, die gebildet wird aus Ammoniak, 2- Aminoethanol, 2- Amino- 2- methylpropan- 1- ol, 2- Amino- 2- methylpropan- 1, 3- diol, Kaliumhydroxid, Natriumhydroxid, L- Arginin, D- Arginin, DL- Arginin, N- Methylglucamin, Morpholin, Imidazol und Harnstoff.

Desweiteren können auch auf einen pH-Wert größer als 7 eingestellte Puffersysteme in dem flüssigen kosmetischen Mittel A3 eingesetzt werden.

Als pH-Puffersystem werden erfindungsgemäß solche chemische Verbindungen bzw. eine Kombination aus chemischen Verbindungen angesehen, die in einer Lösung bewirken, dass sich der pH-Wert der Lösung bei Zugabe einer kleinen Menge Säure oder Lauge zu einem Volumen des kosmetischen Trägers nur geringfügig ändert. Diese Änderung ist weniger ausgeprägt, als dies bei einer Zugabe der gleichen Menge an Säure oder Lauge zu einem gleichen Volumen des kosmetischen Trägers ohne pH-Puffersystem der Fall ist.

Solche pH- Puffersysteme sind bevorzugt ausgewählt aus mindestens einem Vertreter aus der Gruppe, die gebildet wird aus Hydrogencarbonat/ Carbonat, Genußsäure (insbesondere Citronensäure) / Monohydrogenphosphat, Genußsäure (insbesondere Citronensäure) / Dihydrogenphosphat, Tris (hydroxymethyl) aminomethan/ Maleinsäure/ NaOH, Tris (hydroxymethyl) aminomethan/ Maleinsäure/KOH, Tris (hydroxymethyl) aminomethan/HCl, Monohydrogenphosphat/ Dihydrogenphosphat, Dihydrogenphosphat/ NaOH, Dihydrogenphosphat/KOH, $H_3BO_3$/KCl/ NaOH, $H_3BO_3$/KCl/KOH, Borat/HCl, Borat/ Halogenid (insbesondere Chlorid wie Kaliumchlorid) / NaOH, Borat/ Halogenid (insbesondere Chlorid wie Kaliumchlorid) /KOH, Puffersystem nach Theorell und Stenhagen, Puffersystem nach McIlvine, Glycin/ NaOH und Glycin/KOH. Besonders bevorzugte pH- Puffersysteme werden ausgewählt aus mindestens einem Vertreter aus der Gruppe, die gebildet wird, aus Tris (hydroxymethyl) amino- methan/ Maleinsäure/ NaOH, Tris (hydroxymethyl) aminomethan/ Maleinsäure/KOH, Tris (hydroxymethyl) aminomethan/HCl, Borat/HCl und $H_3BO_3$/KCl/ NaOH.

Die mit dem Schrägstrich gekennzeichneten pH-Puffersysteme aus obiger Liste stellen Gemische dieser durch den Schrägstrich getrennten Verbindungen dar. Die in der Liste angegebenen anionischen Verbindungen werden in Form deren Salze mit einem korrespondierenden ein- oder mehrwertigen Kation eingesetzt. Bevorzugte Kationen sind Alkalimetallkationen (insbesondere Natrium oder Kalium) und Ammoniumionen. Erfindungsgemäß in den Puffersystemen verwendbare Genußsäuren sind beispielsweise Citronensäure, Weinsäure oder Äpfelsäure bzw. deren Gemische.

Das pH-Puffersystem ist bevorzugt in einer Menge von 0,1 bis 10,0 Gew.-%, besonders bevorzugt von 0,3 bis 5,0 Gew.-%, ganz besonders bevorzugt von 0,5 bis 3,0 Gew.-%, jeweils bezogen auf das Gewicht der Anwendungsmischung aus Mittel A1 und Mittel A2, bzw. aus den Mitteln A1, A2 und A3, in dem anwendungsbereiten Färbemittel enthalten.

Ein weiterer Gegenstand der vorliegenden Erfindung betrifft ein Verfahren zum Färben von keratinhaltigen Fasern, insbesondere menschlichen Haaren, worin ein des ersten Erfindungsgegenstandes auf die keratinhaltigen Fasern aufgebracht, für eine Einwirkzeit, bevorzugt von 5 bis 45 Minuten, insbesondere bevorzugt von 15 bis 30 Minuten, auf der Faser belassen und anschließend wieder ausgespült oder mit einem Shampoo ausgewaschen wird.

Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt.

Insbesondere liegt die Temperatur während der Einwirkzeit von 5 bis 60 Minuten zwischen 10 °C und 40 °C, insbesondere zwischen 20 °C und 38 °C.

Dabei können die Verbindungen gemäß Formel (I) und die CH-aciden Verbindungen der Formel (II), insbesondere deren vorstehend benannte bevorzugte und besonders bevorzugte Vertreter, als farbgebende Komponenten entweder gleichzeitig auf das Haar aufgebracht werden oder aber auch nacheinander, d. h. in einem mehrstufigen

Verfahren, wobei es unerheblich ist, welche der Komponenten zuerst aufgetragen wird. Die fakultativ enthaltenen Ammonium- oder Metallsalze können dabei den Verbindungen mit der Formel (I) oder den CH-aciden Verbindungen der Formel (II) zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Vor der Anwendung des erfindungsgemäßen Mittels in dem erfindungsgemäßen Verfahren kann es wünschenswert sein, die zu färbende keratinhaltige Faser einer Vorbehandlung zu unterziehen. Die zeitliche Abfolge des dazu erforderlichen Vorbehandlungsschritts und der Anwendung des erfindungsgemäßen Mittels muss nicht unmittelbar nacheinander sein, sondern es kann zwischen dem Vorbehandlungsschritt und der Anwendung des erfindungsgemäßen Mittels ein Zeitraum von bis maximal zwei Wochen liegen. Dazu eignen sich mehrere Vorbehandlungsmethoden. Bevorzugt wird die Faser

V1 vor der Anwendung des erfindungsgemäßen Mittels einer Blondierung oder

V2 vor der Anwendung des erfindungsgemäßen Mittels einer oxidativen Färbung

unterzogen.

Entsprechende erfindungsgemäße Verfahren, bei denen die keratinhaltigen Fasern, bevor ein erfindungsgemäßes Färbemittel zur Anwendung kommt, im Rahmen einer Vorbehandlung mit einem Blondiermittel blondiert oder mit einem Oxidationsfärbemittel gefärbt wurden, sind bevorzugt.

Im Rahmen der Vorbehandlung V1 wird die keratinhaltige Faser mit einem Blondiermittel behandelt. Das Blondiermittel enthält neben einem Oxidationsmittel, wie üblicherweise Wasserstoff-peroxid, bevorzugt mindestens ein als Oxidations- und Bleichverstärker wirksames anorganisches Persalz, wie z.B. ein Peroxodisulfat von Natrium, Kalium oder Ammonium. Färbungen gemäß des erfindungsgemäßen Verfahrens erhalten durch die Vorbehandlung V1 eine besondere Brillanz und Farbtiefe.

Im Rahmen der Vorbehandlung V2 wird ein Mittel enthaltend vorgenannte Oxidationsfarbstoffvorprodukte als Entwickler- und gegebenenfalls Kupplerkomponenten sowie gegebenenfalls vorgenannte Derivate des Indols bzw. Indolins auf die Faser aufgetragen und nach einer Einwirkzeit gegebenenfalls unter Zusatz von vorgenannten geeigneten Oxidationsmitteln auf dem Haar für 5 bis 45, bevorzugt 10 bis 30 Minuten auf der Keratinfaser belassen. Danach wird das Haar gespült. Durch die anschließende Anwendung des erfindungsgemäßen Mittels kann vorhandenen Oxidationsfärbungen einen neue Farbnuance verliehen werden. Wählt man die Farbnuance des erfindungsgemäßen Mittels in der gleichen Farbnuance der oxidativen Färbung aus, so kann die Färbung vorhandener Oxidationsfärbungen nach dem erfindungsgemäßen Verfahren aufgefrischt werden. Es zeigt sich, dass die Farbauffrischung oder Nuancierung gemäß des erfindungsgemäßen Verfahrens einer Farbauffrischung bzw. Nuancierung allein mit herkömmlichen direktziehenden Farbstoffen in der Farbbrillanz und Farbtiefe überlegen ist.

Enthält das anwendungsbereite Mittel neben den Verbindungen gemäß Formel (I) und den CH-aciden Verbindungen der Formel (II) zusätzlich als Oxidationsmittel Wasserstoffperoxid oder ein wasserstoffperoxidhaltiges Oxidationsmittelgemisch, so liegt der pH-Wert des wasserstoffperoxidhaltigen Haarfärbemittels vorzugsweise in einem pH-Bereich von pH 7 bis pH 11, besonders bevorzugt pH 8 bis pH 10. Das Oxidationsmittel kann unmittelbar vor der Anwendung mit dem Haarfärbemittel gemischt und die Mischung auf das Haar aufgebracht werden. Werden die Verbindungen der Formel I und die Komponente B in einem zweistufigen Verfahren auf das Haar appliziert, ist das Oxidationsmittel in einer der beiden Verfahrensstufen zusammen mit der entsprechenden farbgebenden Komponente anzuwenden. Zu diesem Zweck kann es bevorzugt sein, das Oxidationsmittel mit einer der farbgebenden Komponenten in einem Container zu konfektionieren.

Die Verbindungen gemäß Formel (I) der Komponente (Oxo1) und den CH-aciden Verbindungen gemäß Formel (II) der Komponente (Oxo2) können entweder in getrennten Containern oder gemeinsam in einem Container gelagert werden, entweder in einer flüssigen bis pastösen Zubereitung (wässrig oder wasserfrei) oder als Feststoff, beispielsweise als trockenes Pulver. Werden die Komponenten gemeinsam in einer flüssigen Zubereitung gelagert, so sollte diese zur Verminderung einer Reaktion der Komponenten weitgehend wasserfrei sein und einen sauren pH-Wert besitzen. Werden die Komponenten gemeinsam gelagert, so ist es bevorzugt, diese als Feststoff, insbesondere in Form eines bevorzugt mehrschichtigen Formkörpers, z.B. als Tablette zu konfektionieren. Im Falle der mehrschichtigen Formkörper werden die Verbindungen gemäß Formel (I) der Komponente (Oxo1) in eine Schicht und die CH-aciden Verbindungen gemäß Formel (II) der Komponente (Oxo2) in eine andere Schicht eingearbeitet, wobei zwischen diesen Schichten vorzugsweise eine weitere Schicht als Trennschicht liegt. Die Trennschicht ist frei von Verbindungen der Komponenten (Oxo1) und (Oxo2).

Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen, insbesondere 30°C bis 80°C, Wassers hinzugefügt und eine homogene Mischung hergestellt.

Die Färbungen keratinhaltiger Fasern sind bekanntermaßen Umwelteinflüssen, wie beispielsweise Licht, Reibung oder Waschungen, ausgesetzt und können dadurch an Brillanz und Farbtiefe verlieren. Schlimmstenfalls stellt sich gegebenenfalls eine Nuancenverschiebung der Färbung ein. Solche gealterten Färbungen keratinhaltiger Fasern können, wenn der Anwender es wünscht, durch eine Farbauffrischung wieder annähernd in den farblichen Zustand versetzt werden, wie er sich unmittelbar nach der ursprünglichen Färbung präsentierte. Es ist erfindungsgemäß, für eine solche Farbauffrischung eine Kombination aus einer Komponente (Oxo1), enthaltend mindestens eine Verbindung gemäß Formel (I), und aus einer Komponente (Oxo2), enthaltend mindestens eine CH-acide Verbindung der Formel (II), zu verwenden.

Ein weiterer Gegenstand der Erfindung ist daher die Verwendung eines Mittels gemäß erstem Erfindungsgegenstand zur Farbauffrischung von mit oxidativen Färbemitteln gefärbten keratinhaltigen Fasern.

Bezüglich weiterer bevorzugter Ausführungsformen der erfindungsgemäßen Verfahren bzw. der erfindungsgemäßen Verwendung gilt mutatis mutandis das zu den erfindungsgemäßen Mitteln Gesagte.

Die nachfolgenden Beispiele sollen die Erfindung beispielhaft darstellen, ohne sie jedoch darauf zu beschränken und im Schutzumfang einzuschränken.

## Beispiele

### 1.Synthesebeispiele

<u>1.1. Darstellung von 2-Chlor-3,4-dihydroxybenzaldehyd (A1) (erfindungsgemäß)</u>

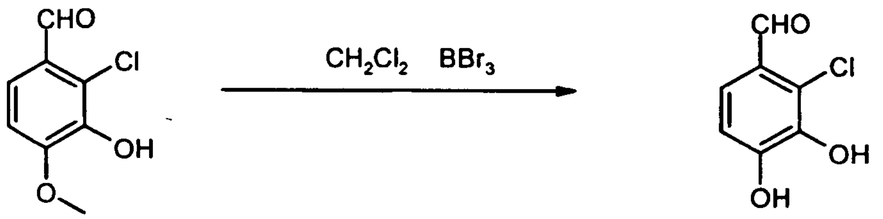


Es wurden 10, 0 g (0, 053 mol) 2- Chlor- 3- hydroxy- 4- methoxybenzaldehyd in 40 ml Dichlormethan gelöst und bei 0 °C tropfenweise mit 25, 0 g (0, 100 mol) Bortribromid versetzt. Nach Beendigung der Zugabe wurde das Reaktionsgemisch für 3 Stunden bei Raumtemperatur gerührt, vorsichtig mit 100 ml Methanol versetzt und dann komplett im Vakuum eingeengt. Der Rückstand wurde in 300 ml Methyl- *tert*- butylether gelöst und mehrmals mit Wasser gewaschen. Die organische Phase wurde getrocknet und komplett eingeengt, als Rückstand resultierte ein beiger Feststoff.
Ausbeute: 7, 5 g (80, 6 %)
$^1$H- NMR (400 MHz, DMSO- $d_6$) : δ [ppm] = 6, 90 (d, 1H) ; 7, 34 (d, 1H) ; 10, 16 (s, 1H)
$^{13}$C- NMR (400 MHz, DMSO- $d_6$) : δ [ppm] = 113, 5; 121, 6; 123, 8; 124, 7; 142, 3; 152, 7; 188, 9

<u>1.2. Darstellung von 2-Brom-3,4-dihydroxyrbenzaldehyd (A2) (erfindungsgemäß)</u>

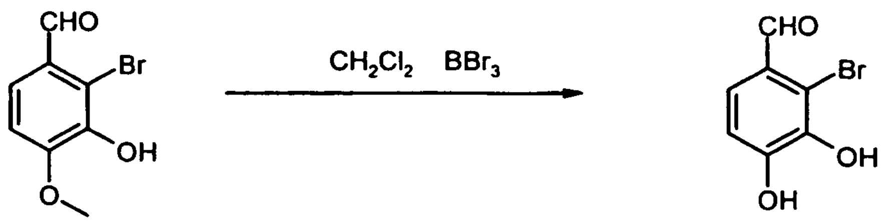


Es wurden 8, 3 g (0, 032 mol) 2- Brom- 3- hydroxy- 4- methoxybenzaldehyd in 40 ml Dichlormethan gelöst und bei 0 °C tropfenweise mit 25, 0 g (0, 100 mol) Bortribromid versetzt. Nach Beendigung der Zugabe wurde das Reaktionsgemisch für 24 Stunden bei Raumtemperatur gerührt, vorsichtig mit 100 ml Methanol versetzt und dann komplett im Vakuum eingeengt. Der Rückstand wurde in 300 ml Methyl- *tert*- butylether gelöst und mehrmals mit Wasser gewaschen. Die organische Phase wurde getrocknet und komplett eingeengt, als Rückstand resultierte ein grauer Feststoff.
Ausbeute: 6, 0 g (75, 9 %)
$^1$H- NMR (400 MHz, DMSO- $d_6$) : δ [ppm] = 6, 93 (d, 1 H) ; 7, 34 (d, 1 H) ; 9, 6 (br., OH) ; 10, 11 (s, 1 H) ; 11, 1 (br., OH)

$^{13}C$- NMR (400 MHz, DMSO- $d_6$) : δ [ppm] = 114, 1; 114, 6; 122, 0; 125, 9; 143, 3; 152, 4; 190, 9

1.3. Darstellung von 5-Chlor-3,4-dihidroxvbenzaldehyd (B3)

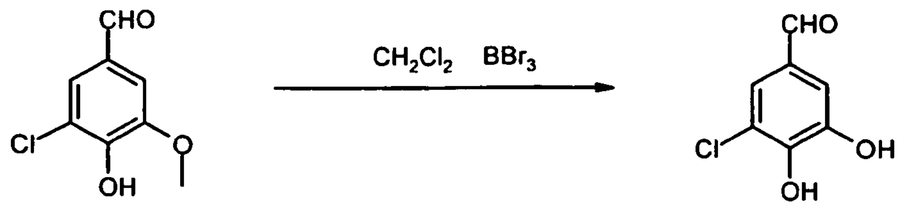


Es wurden 12, 0 g (0, 064 mol) 5- Chlorvanillin (5- Chlor- 4- hydroxy- 3- methoxybenzaldehyd) in 40 ml Dichlormethan gelöst und bei 0 °C tropfenweise mit 25, 0 g (0, 100 mol) Bortribromid versetzt. Nach Beendigung der Zugabe wurde das Reaktionsgemisch für 3 Stunden bei Raumtemperatur gerührt, vorsichtig mit 100 ml Methanol versetzt und dann komplett im Vakuum eingeengt. Der Rückstand wurde in 300 ml Methyl- *tert*- butylether gelöst und mehrmals mit Wasser gewaschen. Die organische Phase wurde getrocknet und komplett eingeengt, als Rückstand resultierte ein beiger Feststoff.
Ausbeute: 8, 8 g (79, 3 %)
$^{1}H$- NMR (400 MHz, DMSO- $d_6$) : δ [ppm] = 7, 24 (s, 1H) ; 7, 46 (s, 1H) ; 9, 73 (s, 1H) ; 10, 5 (br., 2 OH)
$^{13}C$- NMR (400 MHz, DMSO- $d_6$) : δ [ppm] = 112, 2; 120, 2; 124, 1; 128, 4; 147, 0; 148, 5; 190, 7

**2.Färbebeispiele**

**2.1. Herstellung der Zubereitungen**

Alle Mengenangaben sind - soweit nicht anders gekennzeichnet - in Gew.-% angegeben.

2.1.1 Herstellung der Mittel A

Es wurden die folgenden Mittel A, enthaltend eine Verbindung gemäß Formel (I) (Komponente A), hergestellt:

| Rezepturbestandteil | | B1 | B2 |
|---|---|---|---|
| Hydrenol® D | | 7,00 g | 7,00 g |
| Lorol®, techn. | | 2,00 g | 2,00 g |
| Eumulgin® B2 | | 1,00 g | 1,00 g |
| 2-Chlor-3,4-dihydroxybenzaldehyd (erfindungsgemäß) | (B1) | 1,73 g (10 mmol) | - |
| 2-Brom-3,4-dihydroxybenzaldehyd (erfindungsgemäß) | (B2) | - | 2,17 g (10 mmol) |
| Weinsäure / Monoethanolamin | | ad pH 3,8 | ad pH 3,8 |
| Wasser | | ad 100 g | ad 100 g |

Zum Vergleich wurden nicht erfindungsgemäße Mittel bereitgestellt, die halogen- bzw. methoxysubstituierte Brenzcatechinaldehyde enthalten, die nicht der Formel (I) entsprechen:

| Rezepturbestandteil | | B3 | B4 |
|---|---|---|---|
| Hydrenol® D | | 7,00 g | 7,00 g |
| Lorol®, techn. | | 2,00 g | 2,00 g |
| Eumulgin® B2 | | 1,00 g | 1,00 g |
| 5-Chlor-3,4-dihydroxybenzaldehyd (NICHT erfindungsgemäß) | (B3) | 1,73 g (10 mmol) | - |
| 5-Methoxy-3,4-dihydroxybenzaldhehyd (NICHT erfindungsgemäß) | (B4) | - | 1,68 (10 mmol) |
| Weinsäure / Monoethanolamin | | ad pH 3,8 | ad pH 3,8 |
| Wasser | | ad 100 g | ad 100 g |

Hydrenol D, Lorol techn. und Eumulgin B2 wurden zusammen bei 80 °C aufgeschmolzen und anschließend mit 70 g 80 - 90 °C heißem Wasser verrührt. Nach dem Abkühlen auf 50 °C wurde jeweils die angegebene Menge des Benzaldehydderivats, gelöst in 80 - 90 °C heißem Wasser, in diese Fettbasis eingerührt. Unter weiterem Rühren wurde mit Wasser auf ca. 95 g aufgefüllt und der pH-Wert durch Zugabe von Weinsäure bzw. Monoethanolamin auf einen pH-Wert von 3,8 gebracht. Anschließend wurde mit Wasser auf 100 g aufgefüllt und die Cremeformulieruhg komplett kalt gerührt.

2.1.2 Herstellung des Mittels B

Es wurde das folgende Mittel B, enthaltend eine CH-acide Verbindung (Komponente A), hergestellt:

| Rezepturbestandteil | A1 | A2 |
|---|---|---|
| Hydrenol® D | 7,00 g | 7,00 g |
| Lorol®, techn. | 2,00 g | 2,00 g |
| Eumulgin® B2 | 1,00 g | 1,00 g |
| 1,2-Dihydro-1,3,4,6-tetramethyl-2-oxopyrimidiniumhydrogensulfat | 2,50 g (10 mmol) | - |
| 1-Allyl-1,2-dihydro-3,4,6-trimethyl-2-oxopyrimidiniumbromid | - | 2,59 g (10 mmol) |
| Weinsäure / Monoethanolamin | ad pH 1,8 | ad pH 1,8 |
| Wasser | ad 100 g | ad 100 g |

Hydrenol D, Lorol techn. und Eumulgin B2 wurden zusammen bei 80 °C aufgeschmolzen und anschließend mit 70 g 80 - 90 °C heißem Wasser verrührt. Nach dem Abkühlen auf 50 °C wurde die angegebene Menge der C,H-aciden Verbindung, gelöst in 80 - 90 °C heißem Wasser, in diese Fettbasis eingerührt. Unter weiterem Rühren wurde mit Wasser auf ca. 95 g aufgefüllt und der pH-Wert durch Zugabe von Weinsäure bzw. Monoethanolamin auf einen pH-Wert von 1,8 gebracht. Anschließend wurde mit Wasser auf 100 g aufgefüllt und die Cremeformulierung komplett kalt gerührt.

2.2. Ausfärbungen und Färbeergebnisse

Das Mittel A und das Mittel B wurden jeweils im Verhältnis 1:1 vermischt, dann wurde mit Monoethanolamin ein pH-Wert von 8,2 eingestellt. Dieses so erhaltene gebrauchsfertige Haarfärbemittel wurde auf eine Haarsträhne Menschenhaares (Fa. Kerling, naturweiß) aufgebracht (Flottenverhältnis Crememischung / Haare = 3 : 1) und mit einer Applicette gleichmäßig verteilt. Nach einer Einwirkzeit von 30 Minuten bei 32 °C wurde die Strähne ausgespült, mit Shampoo nachgewaschen und danach im warmem Luftstrom getrocknet. Die Färbungen wurden visuell unter einer Tageslichtlampe beurteilt. Dann wurden die Strähnen farbmetrisch mit einem Farbmessgerät der Firma Datacolor, Typ Spectraflash 450, vermessen.

| Formulierung | Nuance / Intensität | L* | a* | b* |
|---|---|---|---|---|
| Ausgangshaar (F. Kerling naturweiß) | - | 75,01 | 2,13 | 21,73 |
| A1 + B1 (erfindungsgemäß) (1,2-Dihydro-1,3,4,6-tetramethyl-2-oxopyrimidiniumhydrogensulfat + 2-Chlor-3,4-dihydroxybenzaldehyd) | schwarz +++ | 18,48 | 3,31 | -0,23 |
| A1 + B2 (erfindungsgemäß) (1,2-Dihydro-1,3,4,6-tetramethyl-2-oxopyrimidiniumhydrogensulfat + 2-Brom-3,4-dihydroxybenzaldehyd) | schwarz +++ | 17,49 | 3,73 | -2,30 |
| A1 + B3 (NICHT erfindungsgemäß) (1,2-Dihydro-1,3,4,6-tetramethyl-2-oxopyrimidiniumhydrogensulfat + 5-Chlor-3,4-dihydroxybenzaldehyd) | schwarzviolett ++ | 18,24 | 7,43 | -4,75 |
| A1 + B4 (NICHT erfindungsgemäß) (1,2-Dihydro-1,3,4,6-tetramethyl-2-oxopyrimidiniumhydrogensulfat + 5-Methoxy-3,4-dihydroxybenzaldhehyd) | schwarzblau ++ | 17,80 | 2,73 | -4,34 |
| A2 + B1 (erfindungsgemäß) (1-Allyl-1,2-dihydro-3,4,6-trimethyl-2-oxopyrimidiniumbromid + 2-Chlor-3,4-dihydroxybenzaldehyd) | schwarz +++ | 18,77 | 3,08 | -5,43 |

(fortgesetzt)

| Formulierung | Nuance / Intensität | L* | a* | b* |
|---|---|---|---|---|
| A2 + B2 (erfindungsgemäß) (1-Allyl-1,2-dihydro-3,4,6-trimethyl-2-oxopyrimidiniumbromid + 2-Brom-3,4-dihydroxybenzaldehyd) | schwarz +++ | 18,48 | 3,31 | -0,23 |
| A2 + B3 (NICHT erfindungsgemäß) (1-Allyl-1,2-dihydro-3,4,6-trimethyl-2-oxopyrimidiniumbromid + 5-Chlor-3,4-dihydroxybenzaldehyd) | schwarzblau ++ | 17,55 | 4,21 | -5,74 |
| A2 + B4 (NICHT erfindungsgemäß) (1-Allyl-1,2-dihydro-3,4,6-trimethyl-2-oxopyrimidiniumbromid + 5-Methoxy-3,4-dihydroxybenzaldehyd) | schwarzblau ++ | 20,90 | -0,84 | -12,22 |
| Farbintensität: +++ = hoch ++.= mittel + = niedrig - = ungefärbt | | | | |

**2.3. Bestimmung der Waschechtheit**

2.3.1. Herstellung von Egalisiersträhnen

Es wurden ca. 15 cm lange, zu 80 % ergraute Haarsträhnen der Firma Kerling verwendet. Der untere Teil der Strähne wurde für 30 Minuten einer Kaltwelle und anschließend für 10 Minuten einer Fixierung unterzogen. Nach dem Auswaschen und Trocknen wurde der untere Strähnenteil für 30 Minuten ultrablondiert, gewaschen und getrocknet. Dann wurde der untere Teil der Haarsträhne nochmals nach obigem Prozedere kaltgewellt und fixiert. Anschließend wurde die gesamte Strähne einer Ultrablondierung unterzogen, gewaschen und nochmals getrocknet. Die so behandelten Haarsträhnen weisen einen starken Schädigungsgrad im unteren Teil der Strähne und eine moderate Schädigung im oberen Teil der Strähne auf.

2.3.2. Ausfärbungen

Jeweils 6,0 g der anwendungsbereiten Färbecreme wurden auf eine Egalisiersträhne aufgetragen und dort für 30 Minuten bei 32 °C belassen. Nach Beendigung der Einwirkzeit wurde das Haar ausgespült und anschließend getrocknet.

2.3.3. Messung von Egalisiervermögen und Waschechtheit

Nach dem Färbevorgang wurden die Egalisiersträhnen farbmetrisch mit einem Farbmessgerät der Firma Datacolor, Typ Spectraflash 450 vermessen. Eine Messung erfolgte im oberen Teil der Haarsträhne, eine weitere Messung wurde im unteren, stark geschädigten Teil der Strähne durchgeführt. Hierbei steht der L-Wert für die Helligkeit (je geringer der L-Wert ist, desto größer ist die Farbintensität), während der a-Wert ein Maß für den Rotanteil einer Farbe ist (d.h. je größer der a-Wert ist, umso größer ist der Rotanteil). Der b-Wert ist ein Maß für den Blauanteil der Farbe, wobei der Blauanteil umso größer ist, je negativer der b-Wert ist Anschließend wurden die Strähnen 6 bzw. 18 mal per Hand mit einer Texapon NSO-UP Lösung gewaschen. Zur Simulation der beim Shampoonieren herrschenden leicht sauren Bedingungen wurde die Texapon-Lösung durch Zugabe von Zitronensäure auf einen pH-Wert von 5 gebracht. Nach jedem Waschvorgang wurden die Strähnen ausgespült und mit einem Föhn getrocknet. Nach dem letzten Trocknen erfolgte eine erneute farbmetrische Vermessung nach dem oben beschriebenen Prozedere. Die Lab-Werte sind in den nachfolgenden Tabellen aufgeführt.

Zur Beurteilung der Waschechtheiten der jeweiligen Nuancen wurde aus den Lab-Werten der dE-Wert berechnet, welcher sich gemäß der nachfolgenden Formel ergibt:

$$dE = [ (L_i - L_0)^2 + (a_i - a_0)^2 + (b_i - b_0)]^{1/2}$$

$L_0$, $a_0$ und $b_0$ sind hierbei die Farbmesswerte vor dem Waschversuch, während $L_i$, $a_i$ und $b_i$ die Farbmesswerte nach dem Waschversuch darstellen. Es wurde jeweils ein dE-Wert für den oberen, moderat geschädigten Teil und den unteren, stark geschädigten Teil einer Egalisiersträhne berechnet.

Nr. 1: Formulierungen A1 + B1 (erfindungsgemäß)

1,2-Dihydro-1,3,4,6-tetramethyl-2-oxopyrimidiniumhydrogensulfat + 2-Chlor-3,4-dihydroxybenzaldehyd

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| VW | 17,78 | 1,27 | -0,16 | | | |
| 6 HW | | | | 17,66 | 1,09 | -0,27 |
| 18 HW | | | | 18,34 | 0,75 | -0,41 |
| Farbnuance | schwarz | | | schwarz | | |
| dE-Wert | 0,24 (6 HW)<br>0,81 (18 HW) | | | | | |
| unterer Strähnenteil | vor der Wäsche (VW) | | | nach 6 (6 HW) und nach 18 (18 HW) Haarwäschen | | |
| | $L_0$ | $a_0$ | $b_0$ | $L_i$ | $a_i$ | $b_i$ |
| VW | 16,34 | 0,56 | -0,31 | | | |
| 6 HW | | | | 17,12 | 0,93 | 0,31 |
| 18 HW | | | | 17,31 | 0,30 | 0,07 |
| Farbnuance | schwarz | | | schwarz | | |
| dE-Wert | 1,06 (6 HW)<br>1,07 (18 HW) | | | | | |

Nr. 2: Formulierungen A1 + B2 (erfindungsgemäß)

1,2-Dihydro-1,3,4,6-tetramethyl-2-oxopyrimidiniumhydrogensulfat + 2-Brom-3,4-dihydroxybenzaldehyd

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| VW | 18,01 | 2,18 | -1,91 | | | |
| 6 HW | | | | 18,29 | 1,35 | -1,47 |
| 18 HW | | | | 18,59 | 0,87 | -0,62 |
| Farbnuance | schwarz | | | schwarz | | |
| dE-Wert | 0,97 (6 HW)<br>1,93 (18 HW) | | | | | |
| unterer Strähnenteil | vor der Wäsche (VW) | | | nach 6 (6 HW) und nach 18 (18 HW) Haarwäschen | | |
| | $L_0$ | $a_0$ | $b_0$ | $L_i$ | $a_i$ | $b_i$ |
| VW | 16,51 | 0,55 | 0,56 | | | |
| 6 HW | | | | 16,42 | 1,19 | 0,45 |
| 18 HW | | | | 16,98 | 0,43 | 0,11 |
| Farbnuance | schwarz | | | schwarz | | |
| dE-Wert | 0,66 (6 HW)<br>0,67 (18 HW) | | | | | |

Nr. 3: Formulierungen A1 + B3 (NICHT erfindungsgemäß)

1,2-Dihydro-1,3,4,6-tetramethyl-2-oxopyrimidiniumhydrogensulfat + 5-Chlor-3,4-dihydroxybenzaldehyd

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| VW | 20,11 | 90,88 | -5,12 | | | |
| 6 HW | | | | 21,87 | 7,64 | -5,02 |

(fortgesetzt)

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| 18 HW<br>Farbnuance | schwarzviolett | | | 22,41<br>mittelbraun | 6,09 | -4,33 |
| dE-Wert | 2,85 (6 HW)<br>4,50 (18 HW) | | | | | |
| unterer Strähnenteil | vor der Wäsche (VW) | | | nach 6 (6 HW) und nach 18 (18 HW) Haarwäschen | | |
| | $L_0$ | $a_0$ | $b_0$ | $L_i$ | $a_i$ | $b_i$ |
| VW<br>6 HW<br>18 HW<br>Farbnuance | 17,88<br><br><br>schwarzviolett | 5,63 | -3,55 | <br>27,58<br>34,51<br>graubraun | <br>4,53<br>1,80 | <br>-2,11<br>3,66 |
| dE-Wert | 9,86 (6 HW)<br>18,52 (18 HW) | | | | | |

Nr. 4: Formulierungen A1 + B4 (NICHT erfindungsgemäß)

1,2-Dihydro-1,3,4,6-tetramethyl-2-oxopyrimidiniumhydrogensulfat + 5-Methoxy-3,4-dihydroxybenzaldhehyd

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| VW<br>6 HW<br>18 HW<br>Farbnuance | 19,55<br><br><br>schwarzblau | 0,32 | 2,28 | <br>18,02<br>17,90<br>dunkelbraun | <br>1,42<br>1,87 | <br>-0,81<br>-0,32 |
| dE-Wert | 3,62 (6 HW)<br>3,45 (18 HW) | | | | | |
| unterer Strähnenteil | vor der Wäsche (VW) | | | nach 6 (6 HW) und nach 18 (18 HW) Haarwäschen | | |
| | $L_0$ | $a_0$ | $b_0$ | $L_i$ | $a_i$ | $b_i$ |
| VW<br>6 HW<br>18 HW<br>Farbnuance | 18,92<br><br><br>schwarzblau | 1,74 | -0,48 | <br>24,49<br>28,88<br>hellbraun | <br>2,89<br>7,00 | <br>2,27<br>10,62 |
| dE-Wert | 6,31 (6 HW)<br>15,81 (18 HW) | | | | | |

Nr. 5: Formulierungen A2 + B1 (erfindungsgemäß)

1-Allyl-1,2-dihydro-3,4,6-trimethyl-2-oxopyrimidiniumbromid + 2-Chlor-3,4-dihydroxybenzaldehyd

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| VW<br>6 HW<br>18 HW<br>Farbnuance | 17,24<br><br><br>schwarz | 1,26 | -2,06 | <br>16,87<br>17,00<br>schwarz | <br>0,43<br>0,33 | <br>-1,39<br>-1,40 |

(fortgesetzt)

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| dE-Wert | 1,13 (6 HW)<br>1,17 (18 HW) | | | | | |
| unterer Strähnenteil | vor der Wäsche (VW) | | | nach 6 (6 HW) und nach 18 (18 HW) Haarwäschen | | |
| | $L_0$ | $a_0$ | $b_0$ | $L_i$ | $a_i$ | $b_i$ |
| VW | 15,76 | 0,63 | -1,27 | | | |
| 6 HW | | | | 16,24 | 0,19 | -0,44 |
| 18 HW | | | | 15,74 | 0,07 | -0,63 |
| Farbnuance | schwarz | | | schwarz | | |
| dE-Wert | 1,05 (6 HW)<br>0,85 (18 HW) | | | | | |

Nr. 6: Formulierungen A2 + B2 (erfindungsgemäß)

1-Allyl-1,2-dihydro-3,4,6-trimethyl-2-oxopyrimidiniumbromid + 2-Brom-3,4-dihydroxybenzaldehyd

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| VW | 18,20 | 1,94 | -5,11 | | | |
| 6 HW | | | | 17,52 | 0,69 | -3,45 |
| 18 HW | | | | 18,59 | 0,31 | -1,62 |
| Farbnuance | schwarz | | | schwarz | | |
| dE-Wert | 2,19 (6 HW)<br>3,88 (18 HW) | | | | | |
| unterer Strähnenteil | vor der Wäsche (VW) | | | nach 6 (6 HW) und nach 18 (18 HW) Haarwäschen | | |
| | $L_0$ | $a_0$ | $b_0$ | $L_i$ | $a_i$ | $b_i$ |
| VW | 15,93 | 0,63 | -1,40 | | | |
| 6 HW | | | | 16,49 | 0,18 | -0,54 |
| 18 HW | | | | 16,54 | 0,12 | -0,41 |
| Farbnuance | schwarz | | | schwarz | | |
| dE-Wert | 1,12 (6 HW)<br>1,27 (18 HW) | | | | | |

Nr. 7: Formulierungen A2 + B3 (NICHT erfindungsgemäß)

1-Allyl-1,2-dihydro-3,4,6-trimethyl-2-oxopyrimidiniumbromid + 5-Chlor-3,4-dihydroxybenzaldehyd

| oberer Strähnenteil | vor der Wäsche (VW) | | | nach 6 (6 HW) und nach 18 (18 HW) Haarwäschen | | |
|---|---|---|---|---|---|---|
| | $L_o$ | $a_0$ | $b_0$ | $L_i$ | $a_i$ | $b_i$ |
| VW | 18,82 | 5,85 | -7,34 | | | |
| 6 HW | | | | 19,45 | 2,64 | -7,30 |
| 18 HW | | | | 19,90 | 2,10 | -6,46 |
| Farbnuance | schwarzblau | | | dunkelbraun | | |
| dE-Wert | 3,26 (6 HW)<br>3,99 (18 HW) | | | | | |

(fortgesetzt)

| unterer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| VW<br>6 HW<br>18 HW<br>Farbnuance | 16,92<br><br><br>schwarzblau | 5,10 | -11,22 | <br>21,91<br>25,81<br>graugrün | <br>0,12<br>-0,27 | <br>-7,21<br>-0,35 |
| dE-Wert | 8,11 (6 HW)<br>15,03 (18 HW) | | | | | |

Nr. 8: Formulierungen A2 + B4 (NICHT erfindungsgemäß)

1-Allyl-1,2-dihydro-3,4,6-trimethyl-2-oxopyrimidiniumbromid + 5-Methoxy-3,4-dihydroxybenzaldhehyd

| oberer Strähnenteil | vor der Wäsche (VW) | | | nach 6 (6 HW) und nach 18 (18 HW) Haarwäschen | | |
|---|---|---|---|---|---|---|
| | $L_0$ | $a_0$ | $b_0$ | $L_i$ | $a_i$ | $b_i$ |
| VW<br>6 HW<br>18 HW<br>Farbnuance | 17,28<br><br><br>schwarzblau | 1,58 | -2,92 | <br>17,60<br>17,96<br>schwarzbraun | <br>0,69<br>1,03 | <br>-1,69<br>-1,33 |
| dE-Wert | 1,55 (6 HW)<br>1,81 (18 HW) | | | | | |
| unterer Strähnenteil | vor der Wäsche (VW) | | | nach 6 (6 HW) und nach 18 (18 HW) Haarwäschen | | |
| | $L_0$ | $a_0$ | $b_0$ | $L_i$ | $a_i$ | $b_i$ |
| VW<br>6 HW<br>18 HW<br>Farbnuance | 16,21<br><br><br>schwarzblau | 1,07 | -2,87 | <br>16,81<br>16,97<br>dunkelbraun | <br>0,93<br>2,13 | <br>0,00<br>0,75 |
| dE-Wert | 2,90 (6 HW)<br>3,80 (18 HW) | | | | | |

2.3.4. Deutung der Ergebnisse

Eine gute Waschechtheit liegt vor, wenn die vor dem Auswaschvorgang erhaltenen L-Werte, a-Werte und b-Werte nur geringfügig von den nach dem Auswaschvorgang erhaltenen Werten abweichen. Die Lab-Werte fließen in die Formel zur Berechnung des dE-Wertes (des Farbabstandes) ein. Eine gute Waschechtheit liegt demzufolge dann vor, wenn der dE-Wert besonders klein ist.

Es ist zu beobachten, daß die dE-Werte bei allen erfindungsgemäßen Kombinationen sowohl im moderat geschädigten oberen Teil des Haares als auch insbesondere im stark geschädigten unteren Teil des Haares klein sind. Bei den Vergleichsversuchen weisen die stark geschädigten Haarpartien große dE-Werte auf, die Waschechtheiten der Färbungen mit den Vergleichsrezepturen sind demzufolge auf stark geschädigtem Haar als schlecht einzustufen.

**3. Verzeichnis der verwendeten Handelsprodukte**

| | |
|---|---|
| Eumulgin® B2 | Cetylstearylalkohol mit ca. 20 EO-Einheiten (INCI-Bezeichnung: Ceteareth-20) (Cognis) |
| Hydrenol® D | $C_{16-18}$-Fettalkohol (INCI-Bezeichnung: Cetearyl alcohol) (Cognis) |
| Lorol® tech. | $C_{12-18}$-Fettalkohol (INCI-Bezeichnung: Coconut alcohol) (Cognis) |

**Patentansprüche**

1. Mittel zur Färbung keratinhaltiger Fasern, insbesondere menschlicher Haare, enthaltend in einem kosmetischen Träger

- mindestens eine reaktive Carbonylverbindung der Formel (I)

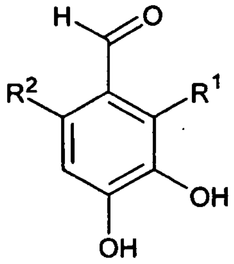

(I)

worin

genau einer der beiden Reste aus $R^1$ und $R^2$ für ein Wasserstoffatom steht und
genau einer der beiden Reste aus $R^1$ und $R^2$ für ein Halogenatom (insbesondere für Chlor, Fluor, Brom oder Iod) steht,

und
- mindestens eine CH- acide Verbindung der Formel (II)

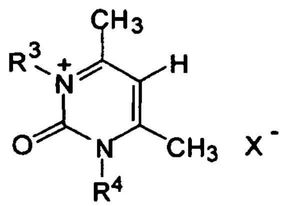

(II)

worin

• $R^3$ und $R^4$ stehen unabhängig voneinander für eine lineare oder cyclische ($C_1$ bis $C_6$)- Alkylgruppe, eine ($C_2$ bis $C_6$)- Alkenylgruppe, eine gegebenenfalls substituierte Arylgruppe, eine gegebenenfalls substituierte Heteroarylgruppe, eine Aryl- ($C_1$ bis $C_6$)- alkylgruppe, eine ($C_1$ bis $C_6$)- Hydroxyalkylgruppe, eine ($C_2$ bis $C_6$)- Polyhydroxyalkylgruppe, eine ($C_1$ bis $C_6$)- Alkoxy- ($C_1$ bis $C_6$)- alkylgruppe, eine Gruppe $R^IR^{II}N-(CH_2)_m-$, worin $R^I$ und $R^{II}$ stehen unabhängig voneinander für ein Wasserstoffatom, eine ($C_1$ bis $C_4$)- Alkylgruppe, eine ($C_1$ bis $C_4$)- Hydroxyalkylgruppe oder eine Aryl- ($C_1$ bis $C_4$)- alkylgruppe, wobei $R^I$ und $R^{II}$ gemeinsam mit dem Stickstoffatom einen 5-, 6- oder 7- gliedrigen Ring bilden können und m steht für eine Zahl 2, 3, 4, 5 oder 6,
• $X^-$ steht für ein physiologisch verträgliches Anion.

2. Mittel nach Anspruch 1, **dadurch gekennzeichnet, dass** es mindestens eine Verbindung gemäß Formel (I) enthält, in der $R^1$ für ein Halogenatom (insbesondere für Fluor, Chlor, Brom oder Iod, bevorzugt für Brom oder Chlor) und $R^2$ für ein Wasserstoffatom steht.

3. Mittel nach einem der Ansprüche 1 oder 2, **dadurch gekennzeichnet, dass** es mindestens eine Verbindung der Formel (I) enthält ausgewählt aus mindestens einer Verbindung der Gruppe, die gebildet wird aus 2- Brom- 3, 4-dihydroxybenzaldehyd, 2- Chlor- 3, 4- dihydroxybenzaldehyd, 2- Fluor- 3, 4- dihydroxybenzaldehyd, 2- Iod- 3, 4-dihydroxybenzaldehyd, 2- Brom- 4, 5- dihydroxybenzaldehyd, 2- Chlor- 4, 5- dihydroxybenzaldehyd, 2- Fluor- 4, 5-dihydroxybenzaldehyd, 2- Iod- 4, 5- dihydroxybenzaldehyd.

**4.** Mittel nach einem der Ansprüche 1 bis 3, **dadurch gekennzeichnet, dass** gemäß Formel (II) $R^3$ für eine Methylgruppe steht und $R^4$ für eine lineare oder cyclische ($C_1$ bis $C_6$)- Alkylgruppe, eine ($C_2$ bis $C_6$)- Alkenylgruppe, eine gegebenenfalls substituierte Arylgruppe, eine gegebenenfalls substituierte Heteroarylgruppe, eine Aryl- ($C_1$ bis $C_6$)-alkylgruppe, eine ($C_1$ bis $C_6$)- Hydroxyalkylgruppe oder eine ($C_2$ bis $C_6$)- Polyhydroxyalkylgruppe steht.

**5.** Mittel nach einem der Ansprüche 1 bis 4, **dadurch gekennzeichnet, dass** es als CH- acide Verbindung der Formel (II) mindestens eine CH- acide Verbindung gemäß Formel (II- a) enthält

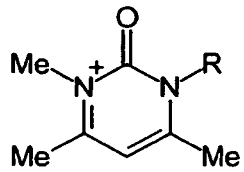

$X^-$ **(II-a)**

wobei

- R für eine Methylgruppe, eine Allylgruppe, eine Hydroxy- ($C_2$- bis $C_6$)- alkylgruppe oder eine gegebenenfalls substituierte Benzylgruppe steht,
- $X^-$ ein physiologisch verträgliches Anion bedeutet.

**6.** Mittel nach Anspruch 5, **dadurch gekennzeichnet, dass** R gemäß Formel (II-a) steht für eine Methylgruppe, eine Allylgruppe, eine 3-Hydroxypropylgruppe, eine 2-Hydroxypropylgruppe, eine 2-Hydroxyethylgruppe oder eine Benzylgruppe.

**7.** Mittel nach einem der Ansprüche 1 bis 6, **dadurch gekennzeichnet, dass** es die Verbindungen mit der Formel (I) in einer Menge von 0,03 bis 65 mmol, insbesondere von 1 bis 40 mmol, bezogen auf 100 g des gesamten Mittels, enthält.

**8.** Mittel nach einem der Ansprüche 1 bis 6, **dadurch gekennzeichnet, dass** es die CH-aciden Verbindungen mit der Formel (II) in einer Menge von 0,03 bis 65 mmol, insbesondere von 1 bis 40 mmol, bezogen auf 100 g des gesamten Mittels, enthält.

**9.** Mittel nach einem der Ansprüche 1 bis 8, **dadurch gekennzeichnet, dass** es einen pH-Wert von größer pH 7 aufweist.

**10.** Kit (Verpackungseinheit), umfassend mindestens ein Mittel A1 und mindestens ein Mittel A2, mit den Maßgaben, dass

- das Mittel A1 in einem kosmetischen Träger mindestens eine CH- acide Verbindung der obigen Formel (II) enthält und
- das Mittel A2 in einem kosmetischen Träger mindestens eine Verbindung der obigen Formel (I) enthält und
- die Mittel A1 und A2 jeweils getrennt konfektioniert vorliegen.

**11.** Verwendung eines Mittels nach einem der Ansprüche 1 bis 9 zur Farbauffrischung von mit oxidativen Färbemitteln gefärbten keratinhaltigen Fasern.

**12.** Verfahren zum Färben von keratinhaltigen Fasern, insbesondere menschlichen Haaren, worin ein Färbemittel nach einem der Ansprüche 1 bis 9 auf die keratinhaltigen Fasern aufgebracht, für eine Einwirkzeit, vorzugsweise von 5 bis 45 Minuten, auf der Faser belassen und anschließend wieder ausgespült oder mit einem Shampoo ausgewaschen wird.

**Claims**

**1.** Agent for colouring keratin-containing fibres, especially human hair, comprising in a cosmetic carrier

- at least one reactive carbonyl compound of Formula (I)

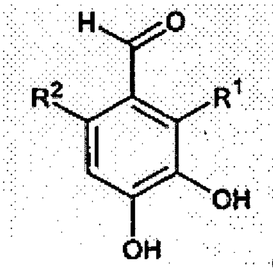

(I)

in which

exactly one of the two residues $R^1$ and $R^2$ stands for a hydrogen atom and
exactly one of the two residues $R^1$ and $R^2$ stands for a halogen atom (in particular for chlorine, fluorine, bromine or iodine),

and
- at least one CH- acidic compound of Formula (II)

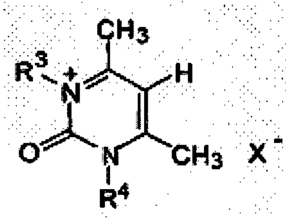

(II)

in which

• $R^3$ and $R^4$ stand independently of one another for a linear or cyclic ($C_1$ to $C_6$) alkyl group, a ($C_2$ to $C_6$) alkenyl group, an optionally substituted aryl group, an optionally substituted heteroaryl group, an aryl ($C_1$ to $C_6$) alkyl group, a ($C_1$ to $C_6$) hydroxyalkyl group, a ($C_2$ to $C_6$) polyhydroxyalkyl group, a ($C_1$ to $C_6$) alkoxy ($C_1$ to $C_6$) alkyl group, a $R^IR^{II}N$- $(CH_2)_m$- group, in which $R^1$ and $R^{II}$ stand independently of one another for a hydrogen atom, a ($C_1$ to $C_4$) alkyl group, a ($C_1$ to $C_4$) hydroxyalkyl group or an aryl ($C_1$ to $C_4$) alkyl group, wherein $R^1$ and $R^{II}$ together with the nitrogen atom can form a 5-, 6- or 7- membered ring and m stands for a number 2, 3, 4, 5 or 6,
• $X^-$ stands for a physiologically compatible anion.

2. Agent according to claim 1, **characterised in that** it comprises at least one compound according to Formula (I), in which $R^1$ stands for a halogen atom (in particular for fluorine, chlorine, bromine or iodine, preferably for bromine or chlorine) and $R^2$ stands for a hydrogen atom.

3. Agent according to one of claims 1 or 2, **characterised in that** it comprises at least one compound of Formula (I) selected from at least one compound of the group consisting of 2- bromo- 3, 4- dihydroxybenzaldehyde, 2- chloro- 3, 4- dihydroxybenzaldehyde, 2- fluoro- 3, 4- dihydroxybenzaldehyde, 2- iodo- 3, 4- dihydroxybenzaldehyde, 2- bromo- 4, 5- dihydroxybenzaldehyde, 2- chloro- 4, 5- dihydroxybenzaldehyde, 2- fluoro- 4, 5- dihydroxybenzaldehyde, 2- iodo- 4, 5- dihydroxybenzaldehyde.

4. Agent according to one of claims 1 to 3, **characterised in that**, according to Formula (II) $R^3$ stands for a methyl group and $R^4$ stands for a linear or cyclic ($C_1$ to $C_6$) alkyl group, a ($C_2$ to $C_6$) alkenyl group, an optionally substituted aryl group, an optionally substituted heteroaryl group, an aryl ($C_1$ to $C_6$) alkyl group, a ($C_1$ to $C_6$) hydroxyalkyl group or a ($C_2$ to $C_6$) polyhydroxyalkyl group.

5. Agent according to one of claims 1 to 4, **characterised in that** it comprises at least one CH-acidic compound according to Formula (II-a) as the compound according to Formula (II)

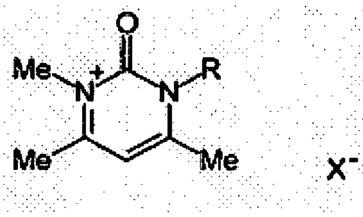

(II-a)

wherein

- R stands for a methyl group, an allyl group, a hydroxy ($C_2$- to $C_6$) alkyl group or an optionally substituted benzyl group,
- $X^-$ means a physiologically compatible anion.

6. Agent according to claim 5, **characterised in that** R according to Formula (II-a) stands for a methyl group, an allyl group, a 3-hydroxypropyl group, a 2-hydroxypropyl group, a 2-hydroxyethyl group or a benzyl group.

7. Agent according to one of the claims 1 to 6, **characterised in that** it comprises the compounds of Formula (I) in an amount of 0.03 to 65 mmol, in particular from 1 to 40 mmol, based on 100 g of the total agent.

8. Agent according to one of the claims 1 to 6, **characterised in that** it comprises the CH-acidic compounds of Formula (II) in an amount of 0.03 to 65 mmol, in particular from 1 to 40 mmol, based on 100 g of the total agent.

9. Agent according to one of the claims 1 to 8, **characterised in that** it exhibits a pH greater than 7.

10. Kit (packaging unit) containing at least one agent A1 and at least one agent A2, with the proviso that

- the agent A1 comprises in a cosmetic carrier at least one CH-acidic compound of the above Formula (II) and
- the agent A2 comprises in a cosmetic carrier at least one compound of the above Formula (I) and
- the agents A1 and A2 are each packaged separately.

11. Use of an agent according to one of claims 1 to 9 for the colour refreshment of keratin-containing fibres that were dyed with oxidative colouring agents.

12. Method for dyeing keratin-containing fibres, especially human hair, in which a colouring agent according to one of claims 1 to 9, is applied onto the keratin-containing fibres, left on the fibres for a contact time, preferably 5 to 45 minutes, and then rinsed out again or washed out with a shampoo.

## Revendications

1. Agent pour la teinture de fibres kératiniques, en particulier de cheveux humains, contenant, dans un support cosmétique :

- au moins un composé carbonyle réactif répondant à la formule (I)

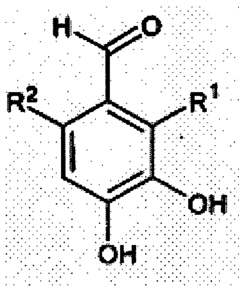

(I)

dans laquelle

précisément un des deux résidus parmi $R^1$ et $R^2$ représente un atome d'hydrogène ; et
précisément un des deux résidus parmi $R^1$ et $R^2$ représente un atome d'halogène (en particulier un atome de chlore, un atome de fluor, un atome de brome ou un atome d'iode) ;

et
- un composé acide CH répondant à la formule (II) :

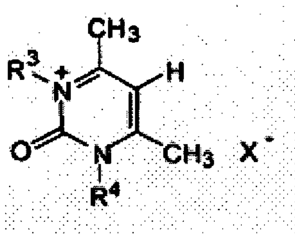

(II)

dans laquelle

• $R^3$ et $R^4$ représentent, indépendamment l'un de l'autre, un groupe alkyle en $C_1$- $C_6$ linéaire ou cyclique, un groupe alcényle en $C_2$- $C_6$, un groupe aryle substitué de manière facultative, un groupe hétéroaryle substitué de manière facultative, un groupe arylalkyle en $C_1$- $C_6$, un groupe hydroxyalkyle en $C_1$- $C_6$, un groupe polyhydroxyalkyle en $C_2$- $C_6$, un groupe alcoxy (en $C_1$- $C_6$) alkyle en $C_1$- $C_6$, un groupe $R^IR^{II}N-(CH_2)_m-$ dans lequel $R^I$ et $R^{II}$ représentent, indépendamment l'un de l'autre, un atome d'hydrogène, un groupe alkyle en $C_1$- $C_4$, un groupe hydroxyalkyle en $C_1$- $C_4$ ou un groupe arylalkyle en $C_1$- $C_4$, $R^I$ et $R^{II}$ pouvant former, ensemble avec l'atome d'azote, un noyau à 5, 6 ou 7 membres, et m représente un nombre égal à 2, 3, 4, 5 ou 6 ;
• X- représente un anion physiologiquement compatible.

2. Agent selon la revendication 1, **caractérisé en ce qu'**il contient au moins un composé répondant à la formule (I) dans laquelle $R^1$ représente un atome d'halogène (en particulier un atome de fluor, un atome de chlore, un atome de brome ou un atome d'iode, de préférence un atome de brome ou un atome de chlore) et $R^2$ représente un atome d'hydrogène.

3. Agent selon l'une quelconque des revendications 1 ou 2, **caractérisé en ce qu'**il contient au moins un composé répondant à la formule (I) choisi parmi au moins un composé du groupe qui est formé par le 2- bromo- 3, 4-dihydroxybenzaldéhyde, le 2- chloro- 3, 4- dihydroxybenzaldéhyde, le 2- fluoro- 3, 4- dihydroxybenzaldéhyde, le 2-iodo- 3, 4- dihydroxybenzaldéhyde, le 2- bromo- 4, 5- dihydroxybenzaldéhyde, le 2- chloro- 4, 5- dihydroxybenzaldéhyde, le 2- fluoro- 4, 5- dihydroxybenzaldéhyde, le 2- iodo- 4, 5- dihydroxybenzaldéhyde.

4. Agent selon l'une quelconque des revendications 1 à 3, **caractérisé en ce que**, conformément à la formule (II), $R^3$ représente un groupe méthyle et $R^4$ représente un groupe alkyle en $C_1$-$C_6$ linéaire ou cyclique, un groupe alcényle en $C_2$-$C_6$, un groupe aryle substitué de manière facultative, un groupe hétéroaryle substitué de manière facultative, un groupe arylalkyle en $C_1$-$C_6$, un groupe hydroxyalkyle en $C_1$-$C_6$ ou un groupe polyhydroxyalkyle en $C_2$-$C_6$.

5. Agent selon l'une quelconque des revendications 1 à 4, **caractérisé en ce qu'**il contient, à titre de composé acide CH répondant à la formule (II), au moins un composé acide CH répondant à la formule (II-a)

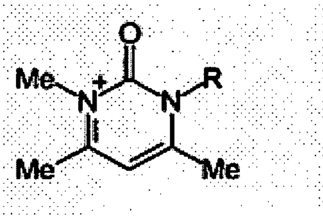

(II-a)

dans laquelle

- R représente un groupe méthyle, un groupe allyle, un groupe hydroxyalkyle en $C_2$--$C_6$ ou un groupe benzyle substitué de manière facultative ;
- X- représente un anion physiologiquement compatible.

**6.** Agent selon la revendication 5, **caractérisé en ce que** R, conformément à la formule (II-a), représente un groupe méthyle, un groupe allyle, un groupe 3-hydroxypropyle, un groupe 2-hydroxypropyle, un groupe 2-hydroxyéthyle ou un groupe benzyle.

**7.** Agent selon l'une quelconque des revendications 1 à 6, **caractérisé en ce qu'**il comprend les composés répondant à la formule (I) en une quantité de 0,03 à 65 mmol, en particulier de 1 à 40 mmol, rapportés à 100 g de l'agent dans sa totalité.

**8.** Agent selon l'une quelconque des revendications 1 à 6, **caractérisé en ce qu'**il contient les composés acides CH répondant à la formule (II) en une quantité de 0,03 à 65 mmol, en particulier de 1 à 40 mmol, rapportés à 100 g de l'agent dans sa totalité.

**9.** Agent selon l'une quelconque des revendications 1 à 8, **caractérisé en ce qu'**il présente une valeur de pH supérieure à 7.

**10.** Kit (unité de conditionnement) comprenant au moins un agent A1 et au moins un agent A2, avec les mesures telles que :

- l'agent A1 contient, dans un support cosmétique, au moins un composé acide CH répondant à la formule (II) indiquée ci-dessus ; et
- l'agent A2 contient, dans un support cosmétique, au moins un composé répondant à la formule (I) indiquée ci-dessus ; et
- les agents A1 et A2 sont présents dans un état confectionné respectivement séparé.

**11.** Utilisation d'un agent selon l'une quelconque des revendications 1 à 9, pour le rafraîchissement des couleurs de fibres kératiniques teintées avec des colorants oxydatifs.

**12.** Procédé pour la teinture de fibres kératiniques, en particulier de cheveux humains, dans lequel on applique sur les fibres kératiniques un colorant selon l'une quelconque des revendications 1 à 9, on le laisse agir sur les fibres pendant un laps de temps, de préférence de 5 à 45 minutes, et on l'en élimine à nouveau par rinçage ou on l'en élimine par lavage avec un shampooing.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1534227 A
- EP 1778176 A
- EP 1789014 A
- WO 2006119819 A
- WO 2007079802 A
- WO 2008074578 A
- EP 998908 A2
- DE 3725030 A
- DE 3723354 A
- DE 3926344 A
- WO 9102047 A


### In der Beschreibung aufgeführte Nicht-Patentliteratur


- **H. BAUMANN.** *Liebigs Ann. Chem.,* 1968, vol. 717, 124-136